# EUROPEAN PATENT APPLICATION

(11) **EP 1 650 223 A2**
(43) Date of publication of application: **26.04.2006**
(21) Application number: 05026068.6
(22) Date of filing: 14.10.2003
(51) Int. Cl.: C07K 14/47, C12N 15/62, G01N 33/53

(54) **RAD51-BRC REPEAT CRYSTALS**

(30) Priority: 14.10.2002 GB 0223860
(62) Divisional of application: 03753806.3
(71) Applicant: CAMBRIDGE UNIVERSITY TECHNICAL SERVICES LIMITED, Cambridge, Cambridgeshire CB2 1TS (GB)
(72) Inventor: Venkitaraman, Ashok, Cambridge, Cambridgeshire CB2 2XZ (GB); Pellegrini, Luca, Cambridge, Cambridgeshire CB2 1GA (GB); Blundell, Tom, Cambridge, Cambridgeshire CB2 1GA (GB); Yu, David, Cambridge, Cambridgeshire CB2 2XZ (GB); Bates, Debbie, Cambridge, Cambridgeshire CB2 2XZ (GB)
(74) Representative: Gill, Stephen Charles

(57) **Abstract**

The structure of a RAD51-BRC repeat sequence complex structure is provided. The structure can be used in modelling the interaction of molecular structures such as potential pharmaceutical compounds. Mutant RAD51 and BRCA2 polypeptides and RAD51-BRC repeat sequence chimaera proteins and are also provided. The mutants may be used in assays for finding compounds which interact with or form part of a RAD51 pathway, and the chimaeras can be used to form crystals which may be analysed by X-ray crystallography.

SEQUENCE LISTING

<110>  Cambridge University Technical Services Ltd

<120>  Polypeptide methods and means

<130>  SCG/CP6344477

<140>

<141>

<150>
<151>

<150>  GB 0223860.8

<151>  2002-10-14

<160>  18

<170>  PatentIn version 3.1

<210>  1

<211>  28

<212>  PRT

<213>  Homo sapiens

<400>  1

Leu Leu Gly Phe His Thr Ala Ser Gly Lys Lys Val Lys Ile Ala Lys
1               5                   10                  15

Glu Ser Leu Asp Lys Val Lys Asn Leu Phe Asp Glu
            20                  25

<210>  2

<211>  26

<212>  PRT

<213>  Artificial sequence

<220>

<223> Consensus

<220>

<221> MISC_FEATURE

<222> (1)..(1)

<223> Xaa = Gly or Ser

<220>

<221> MISC_FEATURE

<222> (3, 11, 18, 22)

<223> Xaa = no preference

<220>

<221> MISC_FEATURE

<222> (4)..(4)

<223> Xaa = Thr or Ser

<220>

<221> MISC_FEATURE

<222> (7)..(7)

<223> Xaa = Gly or Ser or Asn

<220>

<221> MISC_FEATURE

<222> (9, 14, 15, 25)

<223> Xaa = hydrophilic

<220>

<221> MISC_FEATURE

<222> (10, 12)

<223> Xaa = hydrophobic

<220>

<221> MISC_FEATURE

<222> (16)..(16)

<223>  Xaa = Ser or Ala

<220>

<221>  MISC_FEATURE

<222>  (20)..(20)

<223>  Xaa = Ala or Val or Ser

<220>

<221>  MISC_FEATURE

<222>  (21)..(21)

<223>  Xaa = Lys or Arg

<220>

<221>  MISC_FEATURE

<222>  (23)..(23)

<223>  Xaa = hydrophobic or aromatic

<220>

<221>  MISC_FEATURE

<222>  (24)..(24)

<223>  Xaa = Phe or Leu

<220>

<221>  MISC_FEATURE

<222>  (26)..(26)

<223>  Xaa = Asp or Glu

<400>  2

Xaa Phe Xaa Xaa Ala Ser Xaa Lys Xaa Xaa Xaa Xaa Ser Xaa Xaa Xaa
1               5                   10                  15

Leu Xaa Lys Xaa Xaa Xaa Xaa Xaa Xaa Xaa
            20                  25

<210>  3

<211>  7

<212>  PRT

<213>  Homo sapiens

<400>  3

Gly Phe Thr Thr Ala Thr Glu
1               5


<210>  4

<211>  7

<212>  PRT

<213>  Drosophila melanogaster


<400>  4

Gly Phe Leu Ser Ala Arg Thr
1               5


<210>  5

<211>  7

<212>  PRT

<213>  Saccharomyces cerevisiae


<400>  5

Gly Phe Val Thr Ala Ala Asp
1               5


<210>  6

<211>  7

<212>  PRT

<213>  Homo sapiens


<400>  6

Gly Phe Leu Thr Ala Phe Glu
1               5


<210>  7

<211>  7

<212>  PRT

<213>  Pyrococcus furiosus


<400>  7

Thr Phe Met Arg Ala Asp Glu
1               5

<210>  8

<211>  7

<212>  PRT

<213>  Escherichia coli

<400>  8

Ser Ile Met Arg Leu Gly Glu
1               5

<210>  9

<211>  7

<212>  PRT

<213>  Homo sapiens

<400>  9

Gly Phe His Thr Ala Ser Gly
1               5

<210>  10

<211>  12

<212>  PRT

<213>  Artificial sequence

<220>

<223>  Flexible polypeptide linker

<400>  10

Gly Gly Gly Gly Gly Gly Gly Gly Gly Gly Gly Gly
1               5                   10

<210>  11

<211>  12

<212>  PRT

<213>  Artificial sequence

<220>

<223>  Flexible polypeptide linker

<400>  11

Ser Ser Ser Ser Ser Ser Ser Ser Ser Ser Ser Ser
1               5                   10

<210>  12

<211>  12

<212>  PRT

<213>  Artificial sequence

<220>

<223>  Flexible polypeptide linker

<400>  12

Gly Ser Gly Ser Gly Ser Gly Ser Gly Ser Gly Ser
1               5                   10

<210>  13

<211>  7

<212>  PRT

<213>  Artificial sequence

<220>

<223>  Conserved BRC repeat sequence

<220>

<221>  MISC_FEATURE

<222>  (3)..(3)

<223>  Xaa = no preference

<400>  13

Gly Phe Xaa Thr Ala Ser Gly
1               5

<210>  14

<211>  14

<212>  PRT

<213>  Artificial sequence

<220>

<223>  Flexible polypeptide linker

<400>  14

Thr Gly Ser Thr Gly Ser Thr Gly Ser Thr Gly Ser Met Gly
1               5                   10

<210>  15

<211>  5

<212>  PRT

<213>  Homo sapiens

<400>  15

Thr Ala Ser Gly Lys
1               5

<210>  16

<211>  7

<212>  PRT

<213>  Homo sapiens

<400>  16

Phe His Thr Ala Ser Gly Lys
1               5

<210>  17

<211>  8

<212>  PRT

<213>  Homo sapiens

<400>  17

Gly Glu Phe Arg Thr Gly Lys Thr
1               5

<210>  18

<211>  5

<212>  PRT

<213>  Homo sapiens

<400>  18

Leu Leu Ile Val Asp
1               5

## Description

The present invention concerns polypeptide methods and means relating to RAD51, BRCA2 and BRC repeat sequences.

Inheritance of one defective copy of the BRCA2 gene causes increased susceptibility to breast, ovarian and other cancers, with a penetrance approaching 70% by age 70 years ¹. BRCA2 encodes a large protein (3,418 amino acids), which localizes to the nucleus of mitotic cells during S phase of the cell cycle, and is also highly expressed during meiosis. The amino acid sequence of the BRCA2 protein offers few clues to its biological role, because it does not closely resemble other proteins of known function, and has no orthologues in the yeast, fly, or worm genomes.

One remarkable feature of the BRCA2 protein ² is the presence of eight conserved sequence motifs - the BRC repeats - of about 30 amino acids each, positioned between residues 990 to 2940 in human BRCA2. The high degree of conservation between the BRC repeats in different species is particularly striking when compared to the limited overall sequence similarity among BRCA2 orthologs 3,4, suggesting that the BRC motifs perform an essential function in physiological processes where BRCA2 is implicated. Indeed, the BRC repeats are the primary sites through which BRCA2 binds directly to RAD51 5-7, a protein with a crucial role in DNA recombination. Like its bacterial homologue RecA, RAD51 coats single-stranded DNA substrates to form a helical nucleoprotein filament, which can invade duplex DNA and pair with homologous nucleotides to initiate the strand exchange reactions that culminate in genetic recombination. When expressed in vitro 5-7, each of the eight BRC repeats in BRCA2 can interact directly with recombinant RAD51. BRC3 and BRC4 encoded in human BRCA2 are particularly efficient at RAD51 binding, whereas BRC5 and BRC6 are not.

There is growing evidence that the interaction between BRCA2 and RAD51 is critical for the biological functions of both molecules ^{8,9}. Discrete nuclear foci containing RAD51 usually accumulate within the nucleus of mammalian cells exposed to DNA damage. RAD51 foci fail to form in BRCA2-deficient cells 7,10,11, suggesting that BRCA2 transports RAD51 to sites where DNA damage is processed by recombination. Indeed, BRCA2 deficiency leads to a severe defect in the repair of DNA double-s.trand breaks by recombination ¹², and like RAD51 deficiency ^{13,14}, provokes spontaneous instability of chromosome structure during cell division ^{15,16}. Surprisingly - and in apparent conflict with these data - the activity of RAD51 in nucleoprotein filament formation is suppressed by its interaction with peptides encoding BRC repeats 17. Collectively, the experimental evidence suggests models in which the intracellular transport of BRCA2-RAD51 complexes and their activity in nucleoprotein filament formation are regulated following DNA damage, perhaps resulting in transitions from 'inactive' to 'active' states ^{9, 17}.

A major factor holding back further elucidation of RAD51 and BRCA2 functionality and interaction is the lack of determined crystal structures for these proteins. One reason for this is the difficulty, well known in the art, of forming protein crystals having a quality which is sufficiently high to allow the protein structures to be determined by X-ray crystallography. To date, as far as we are aware, no investigators have been able to identify suitable crystallisation procedures for forming BRCA2/RAD51 complex crystals of the required quality.

An additional difficulty associated specifically with RAD51 is the tendency for RAD51 to aggregate in solution. This tendency has defeated previous attempts to crystallise RAD51.

### Disclosure of the Invention

In general aspects, the present invention is concerned with the provision of a RAD51-BRC repeat sequence complex structure and its use e.g. in modelling the interaction of molecular structures such as potential pharmaceutical compounds.

In further general aspects, the present invention is concerned with the provision of mutant RAD51 and BRCA2 polypeptides and preferably a mutant RAD51 polypeptide which has a reduced tendency to aggregate in solution. Such a mutant may be used e.g. in assays for finding compounds which interact with or form part of a RAD51 pathway.

Another general aspect of the present invention concerns a RAD51-BRC repeat sequence chimaera protein. Such a chimaera can be used to form crystals which may be analysed by X-ray crystallography.

These and other aspects and embodiments of the present invention are discussed below.

The above aspects of the invention, both singly and in combination, all contribute to features of the invention which are advantageous.

The present invention is described below in relation to the following figures in which:

### Brief Description of the Drawings

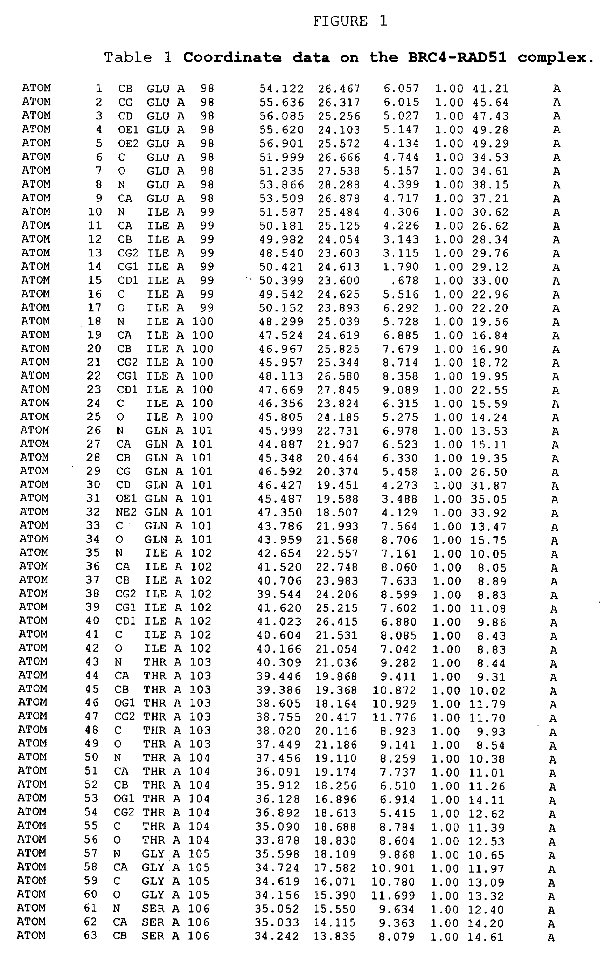

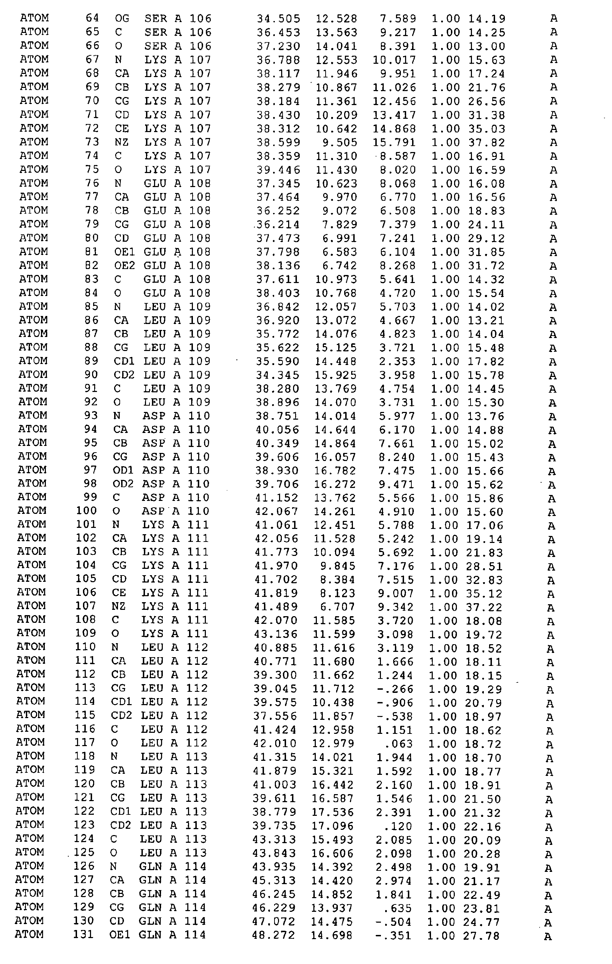

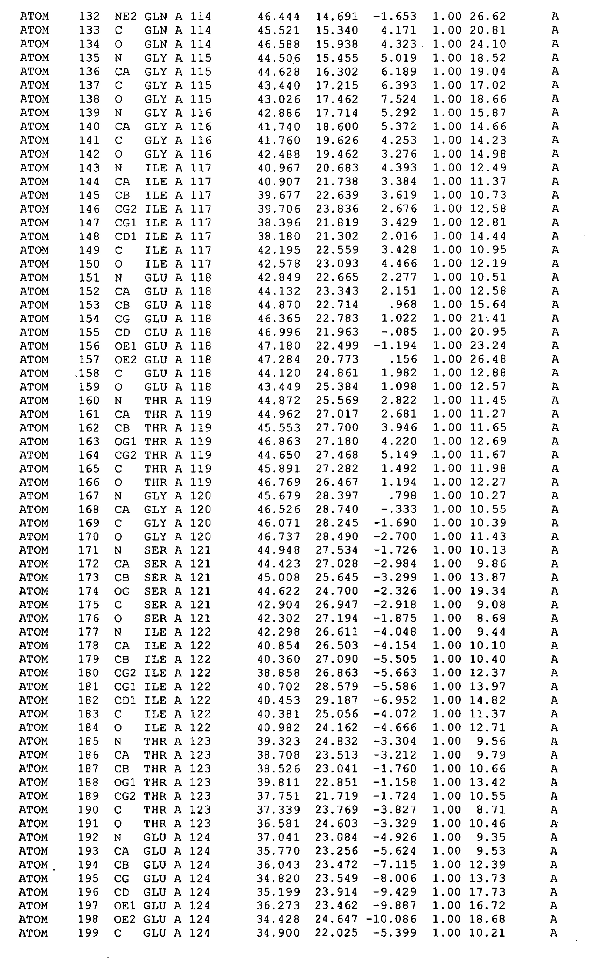

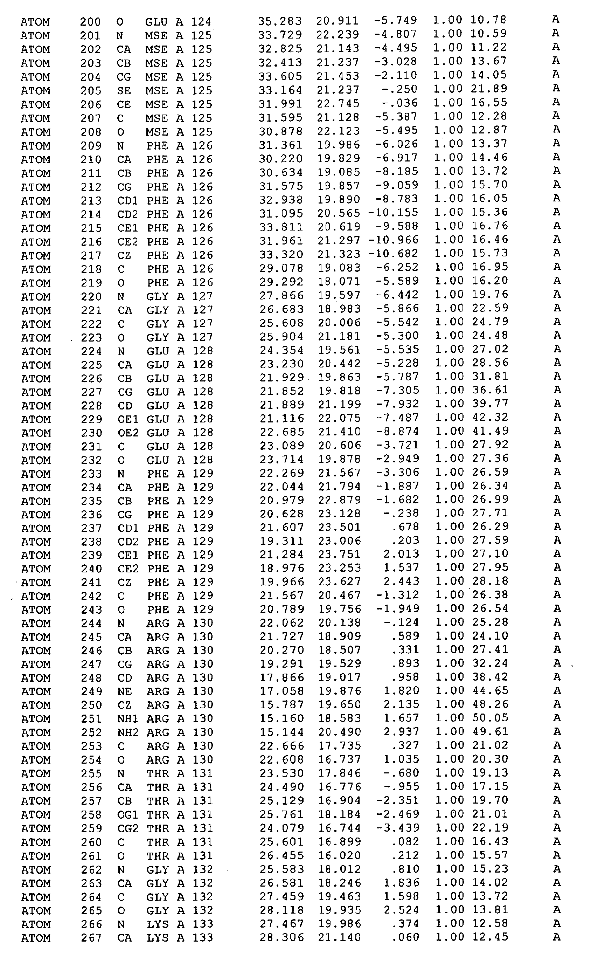

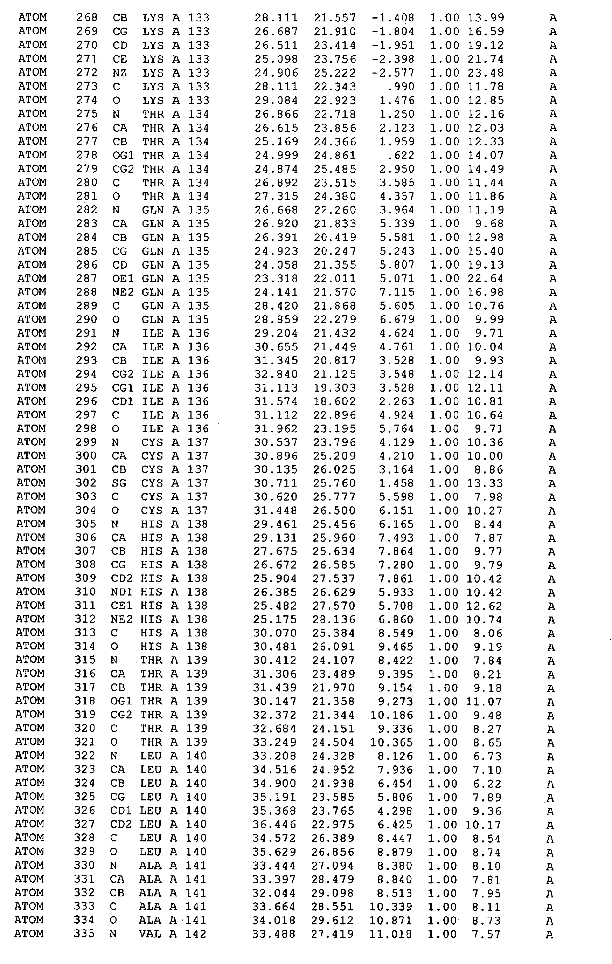

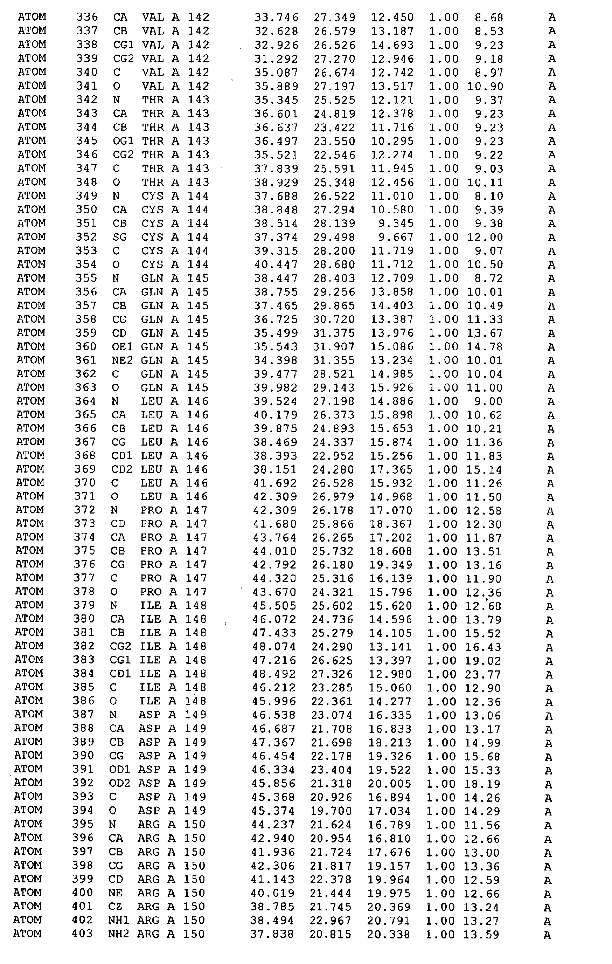

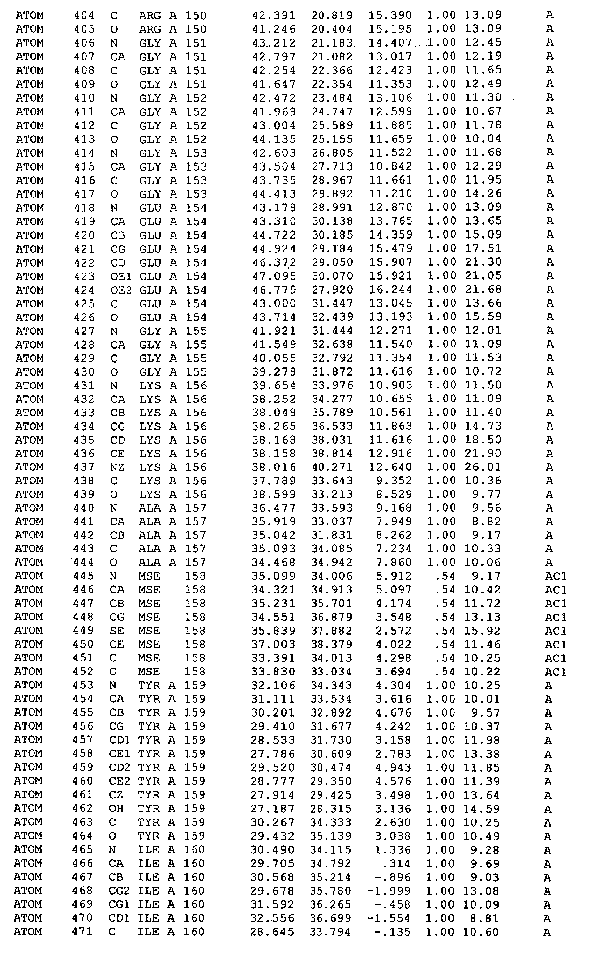

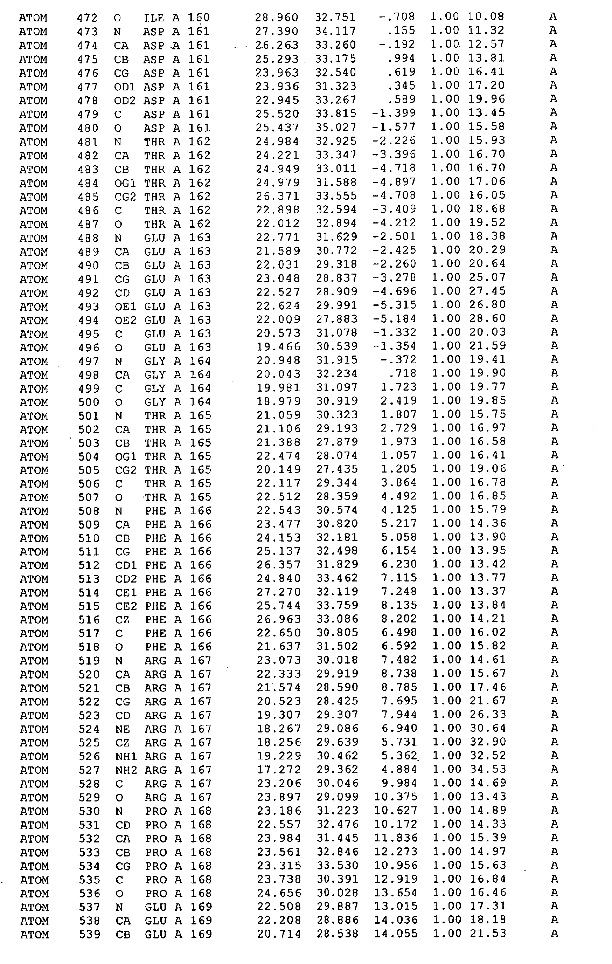

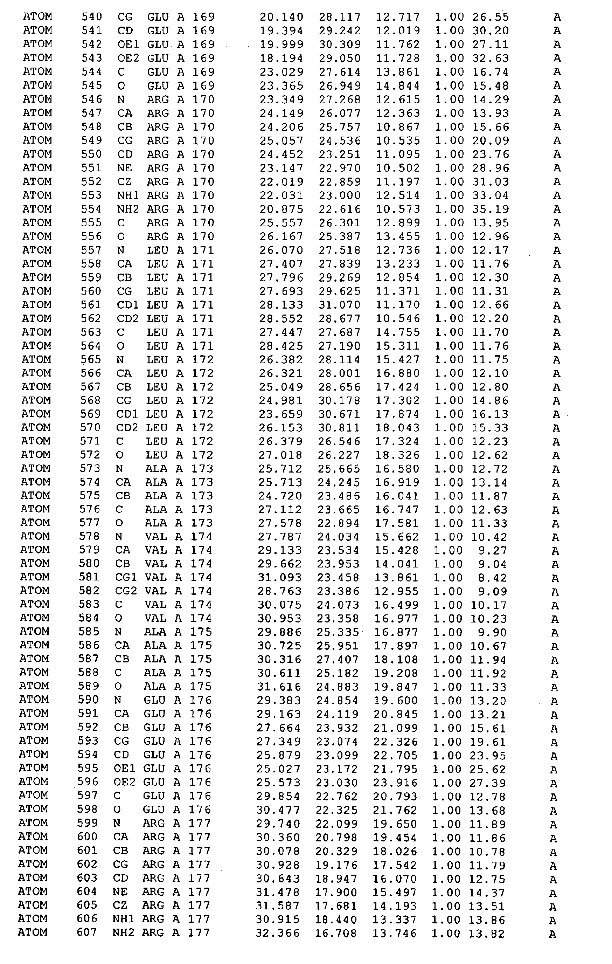

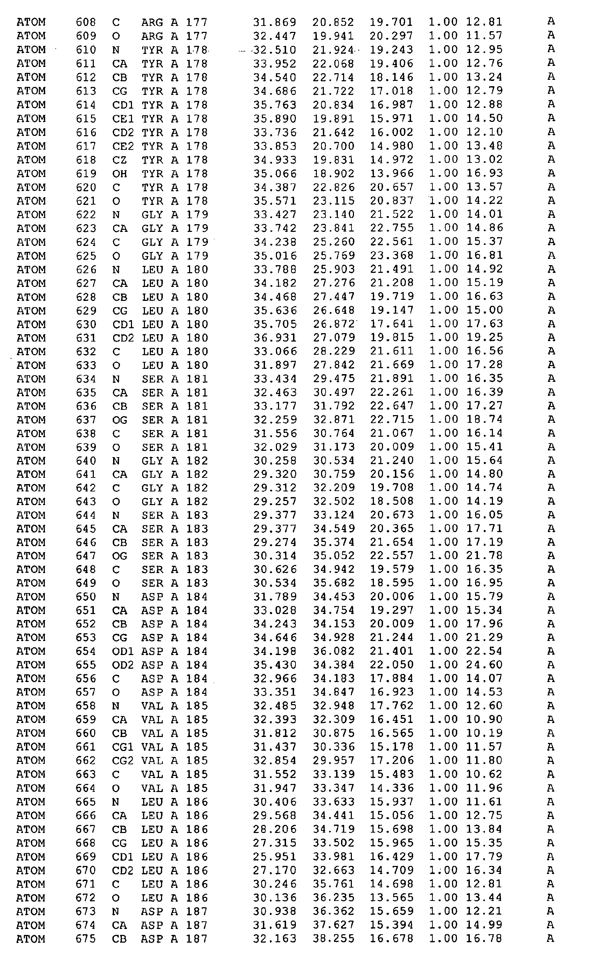

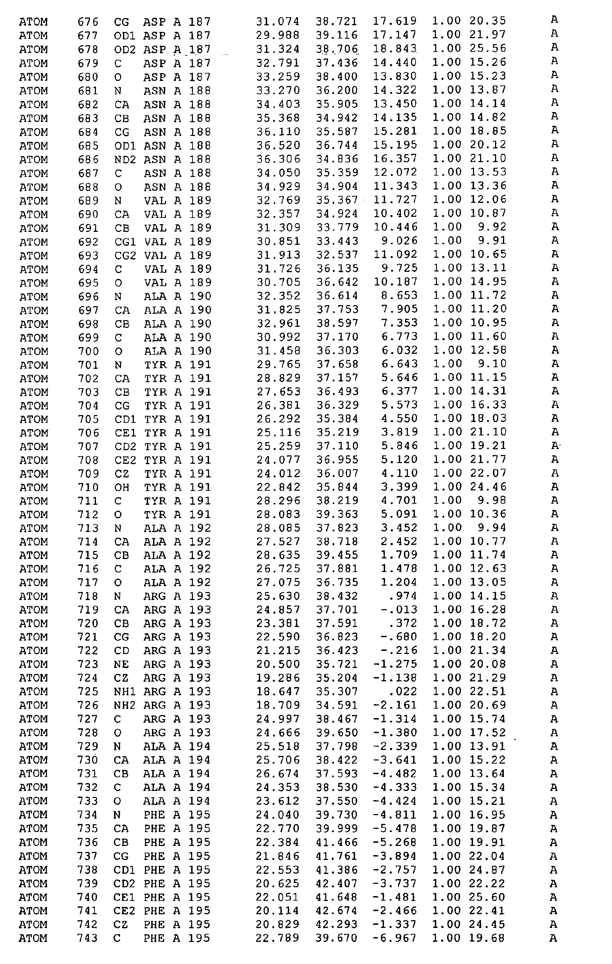

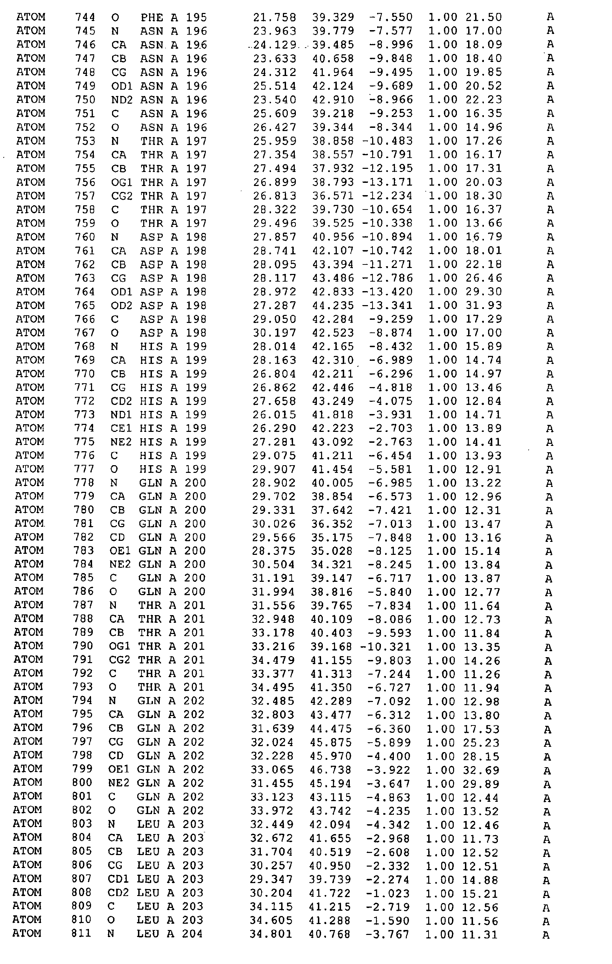

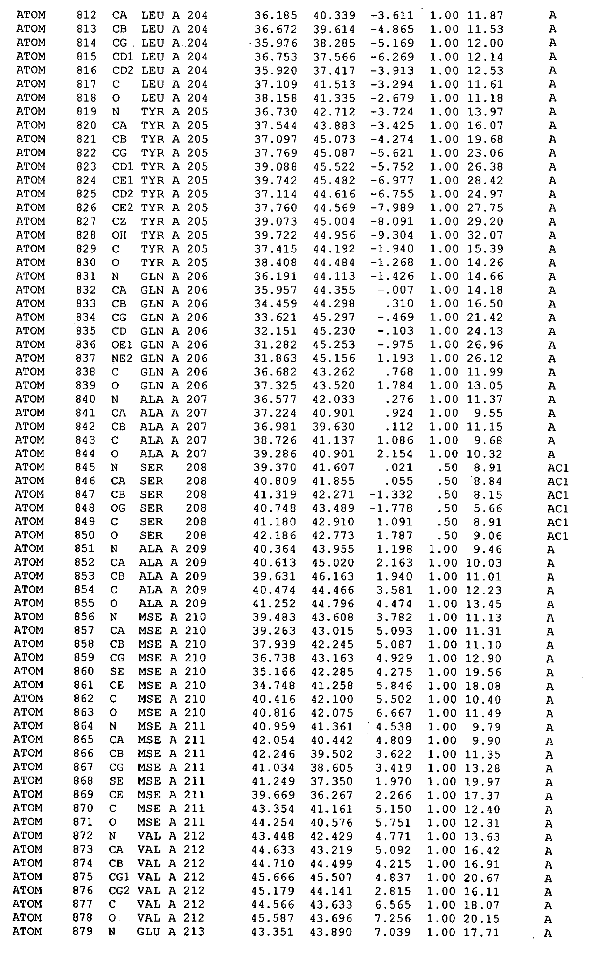

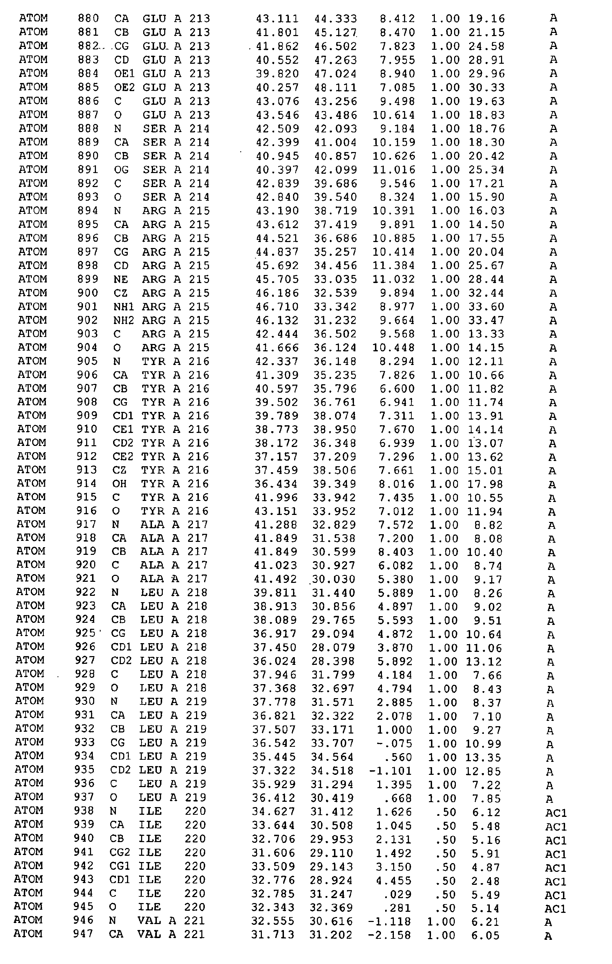

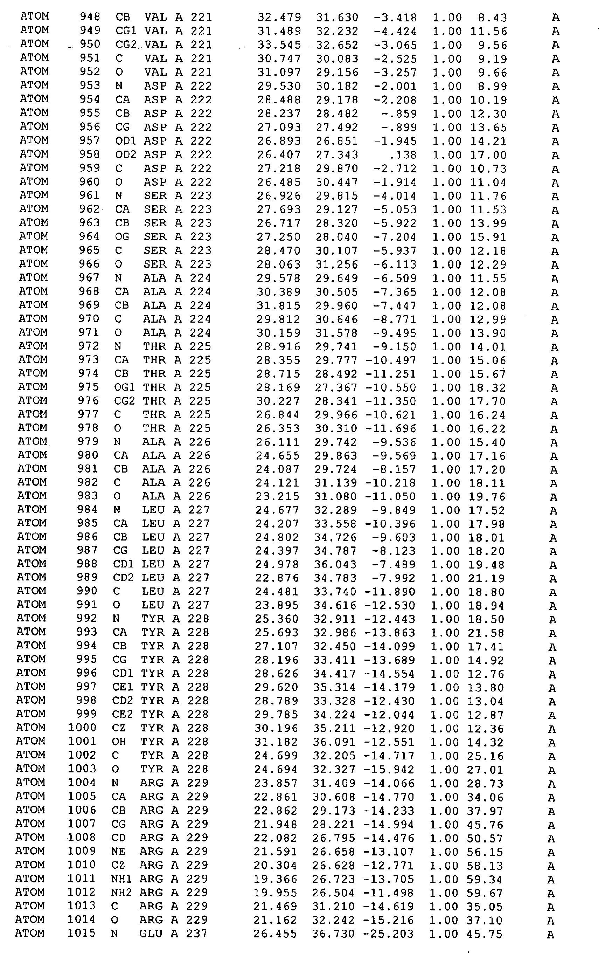

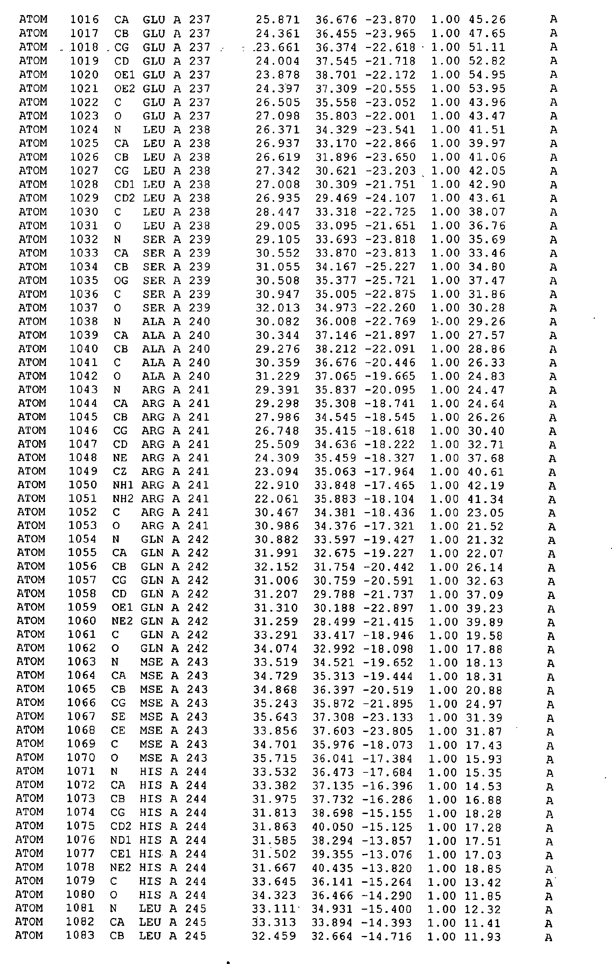

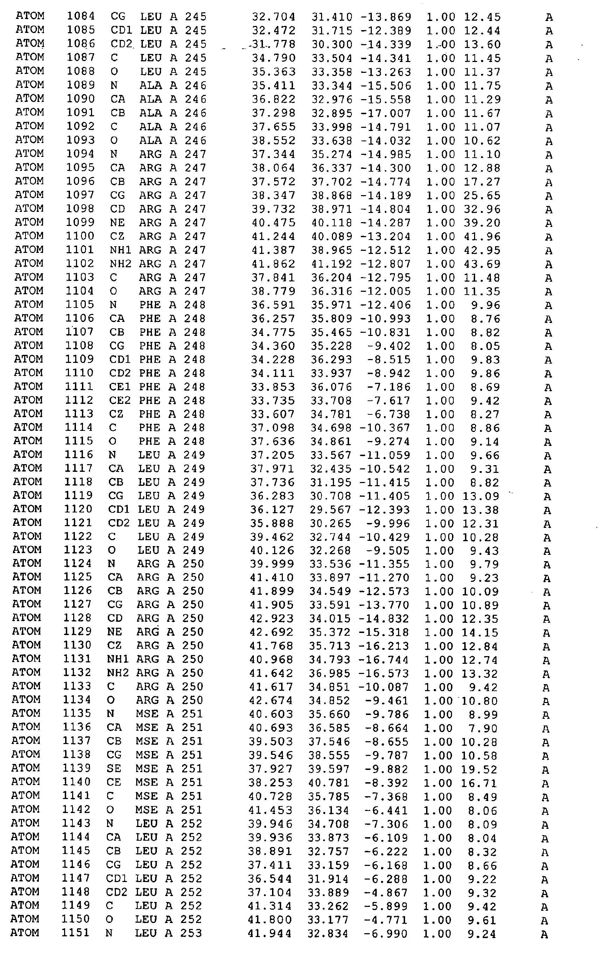

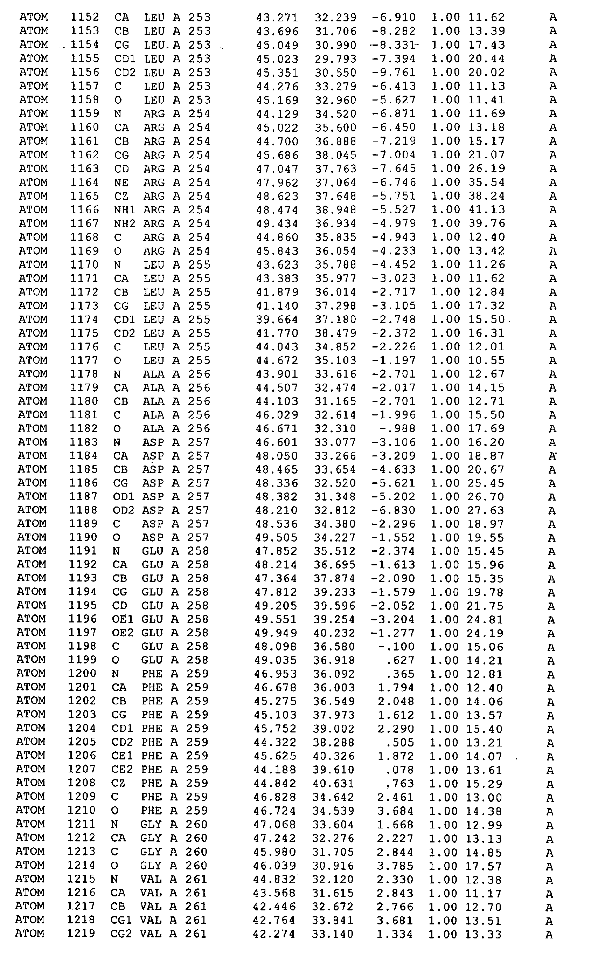

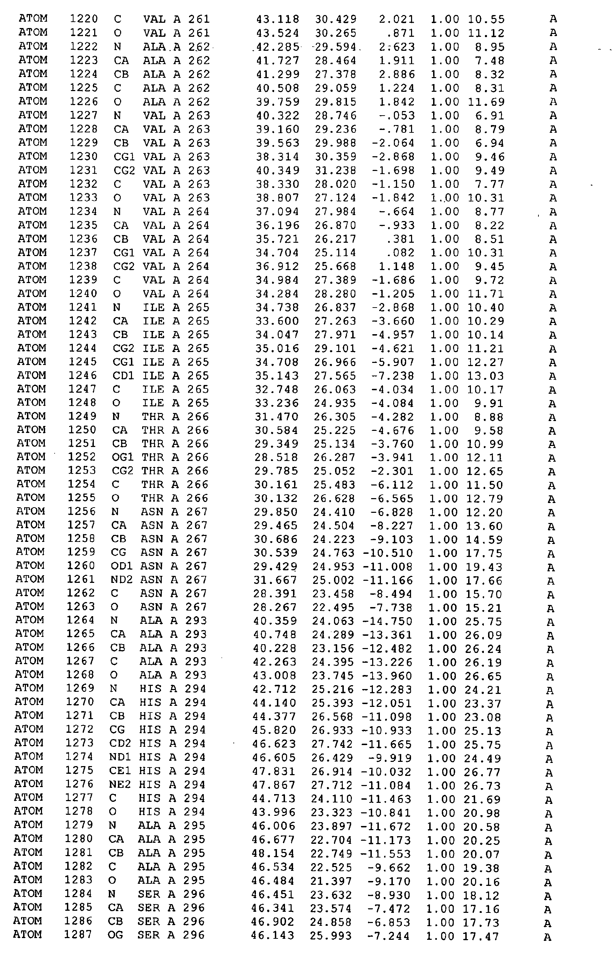

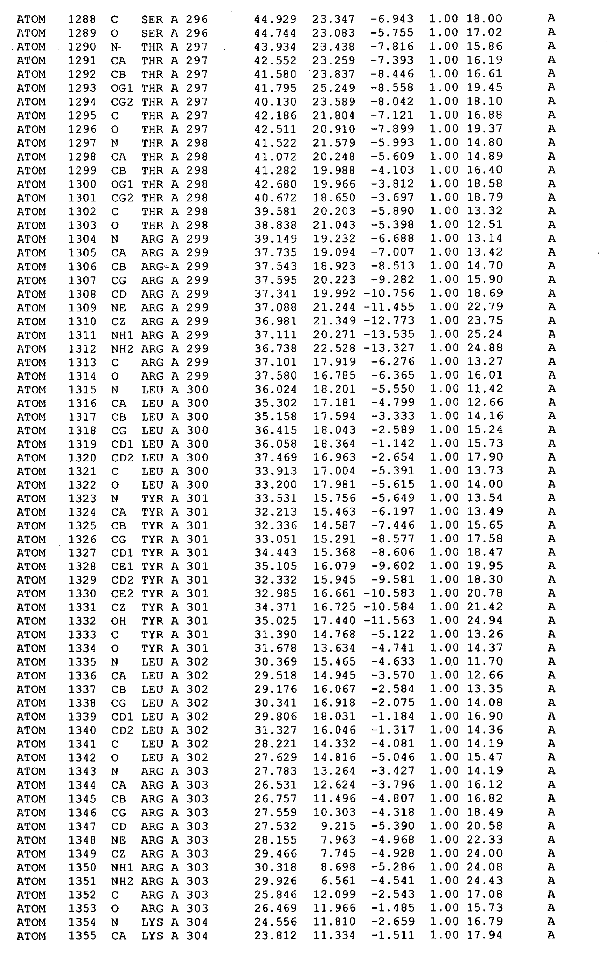

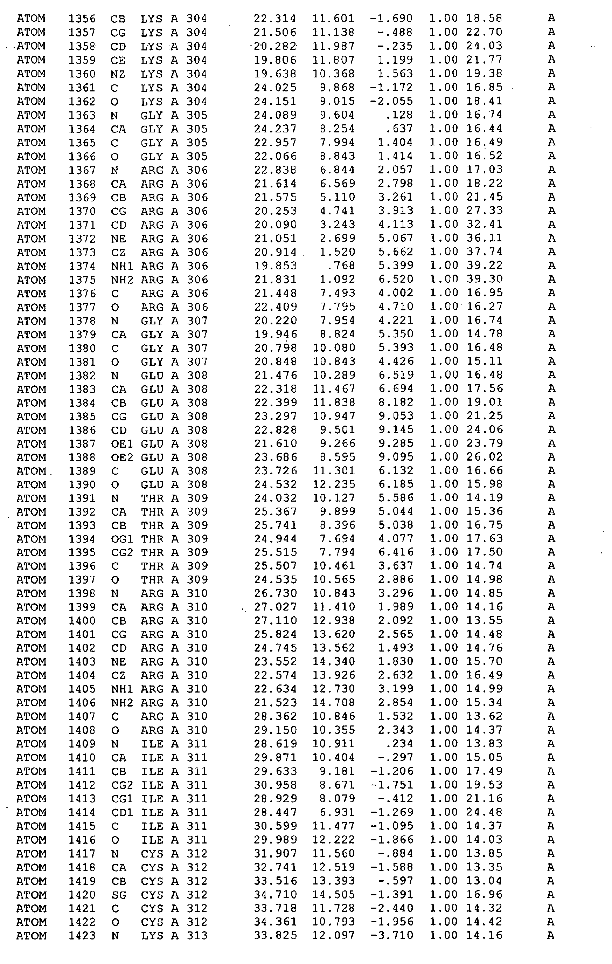

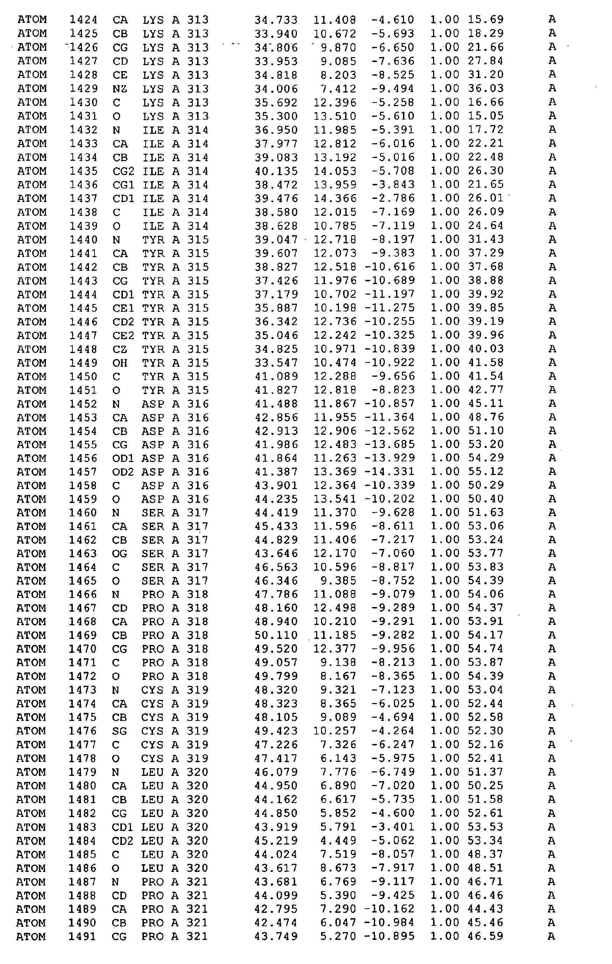

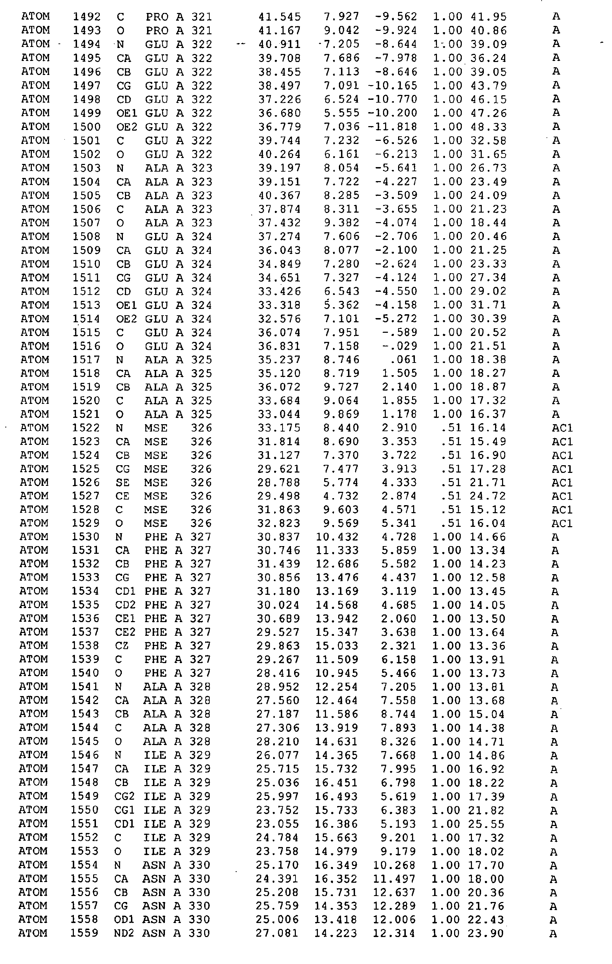

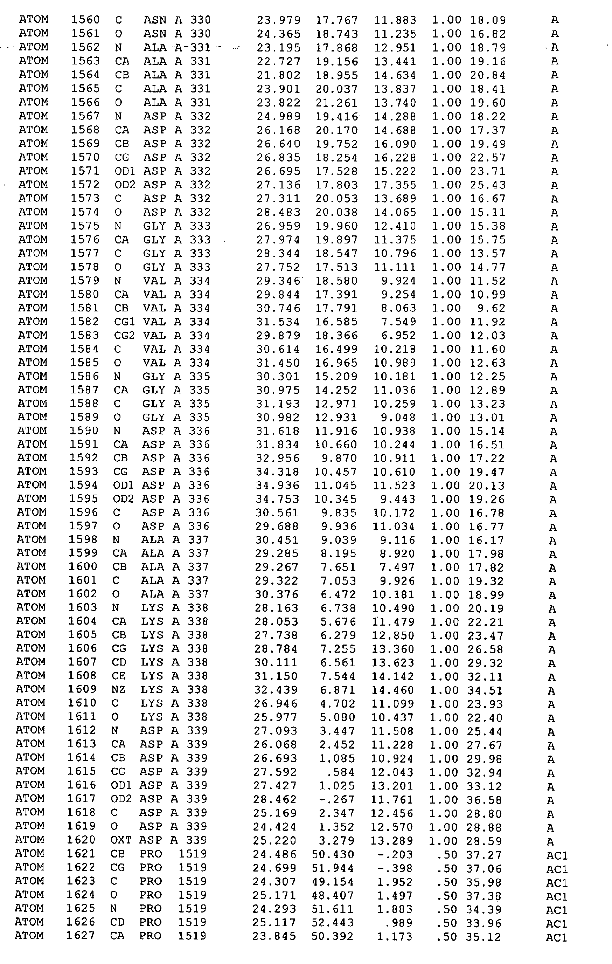

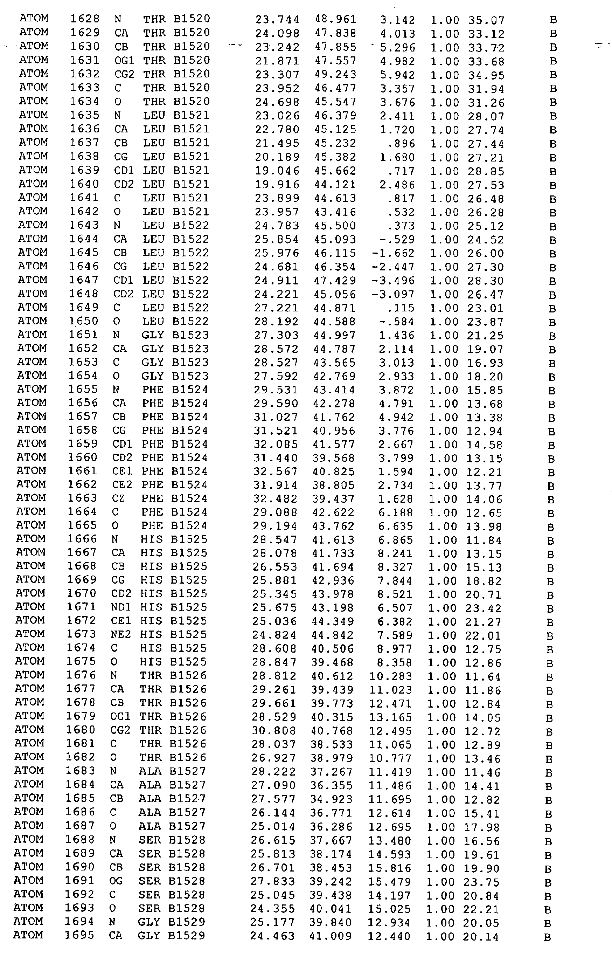

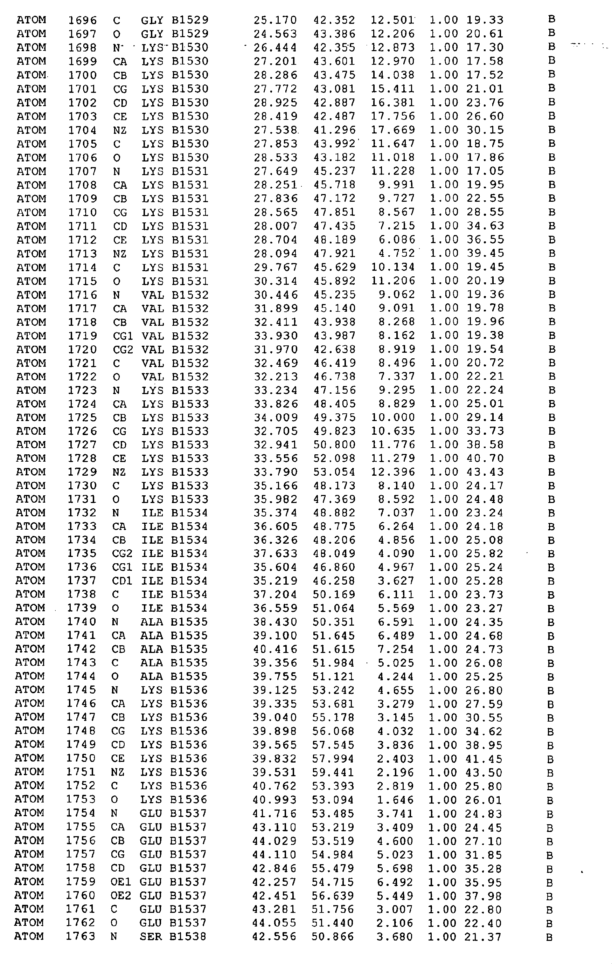

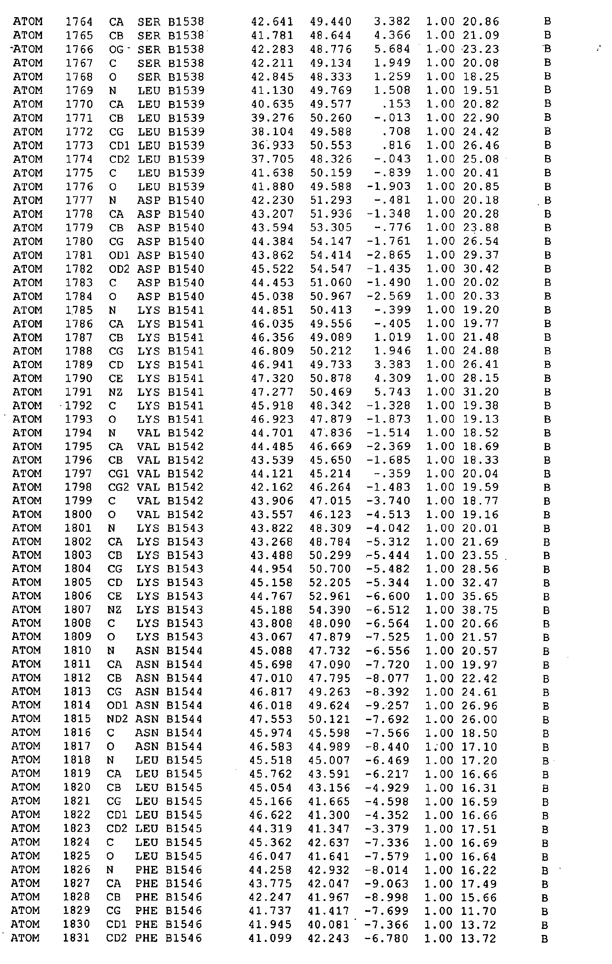

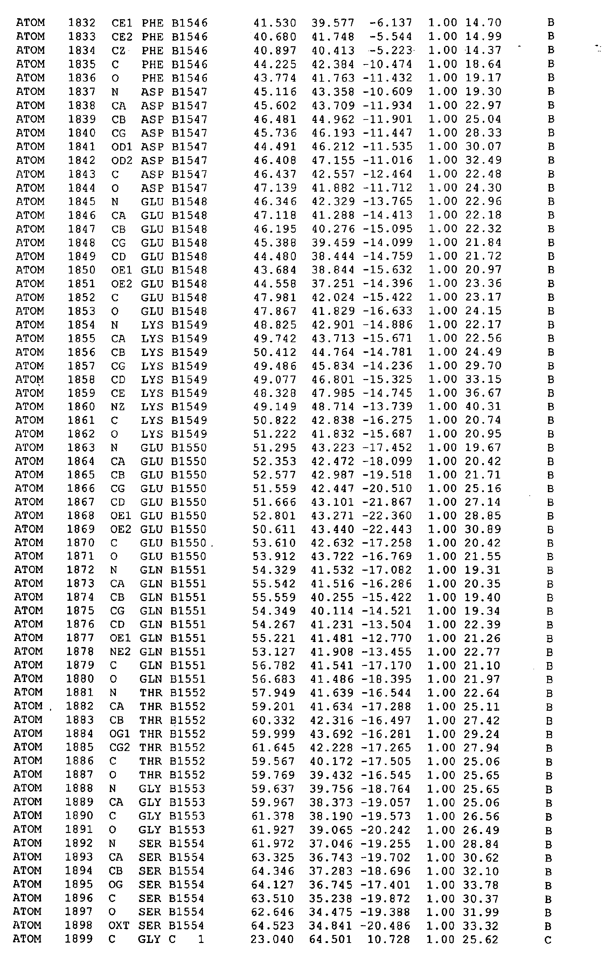

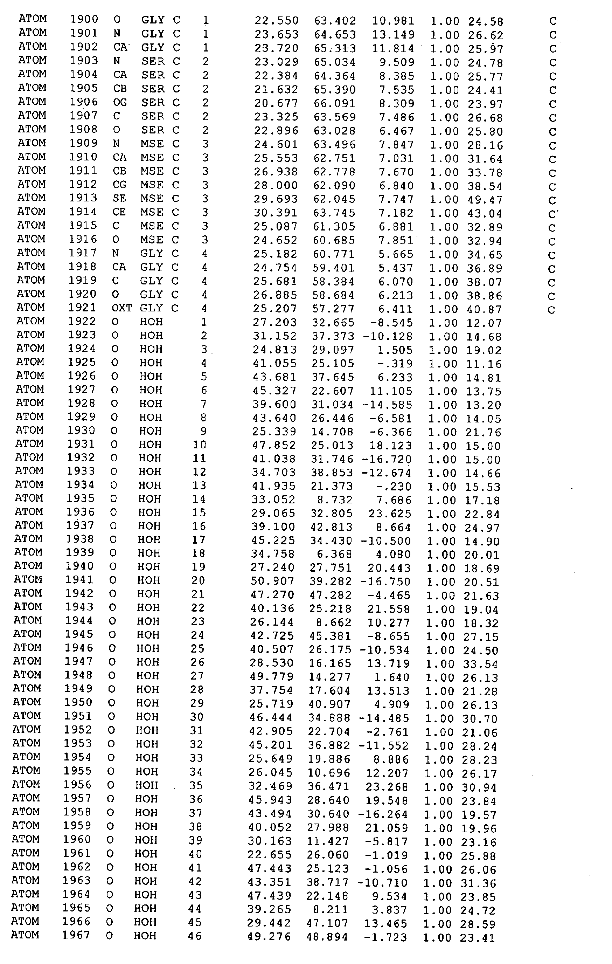

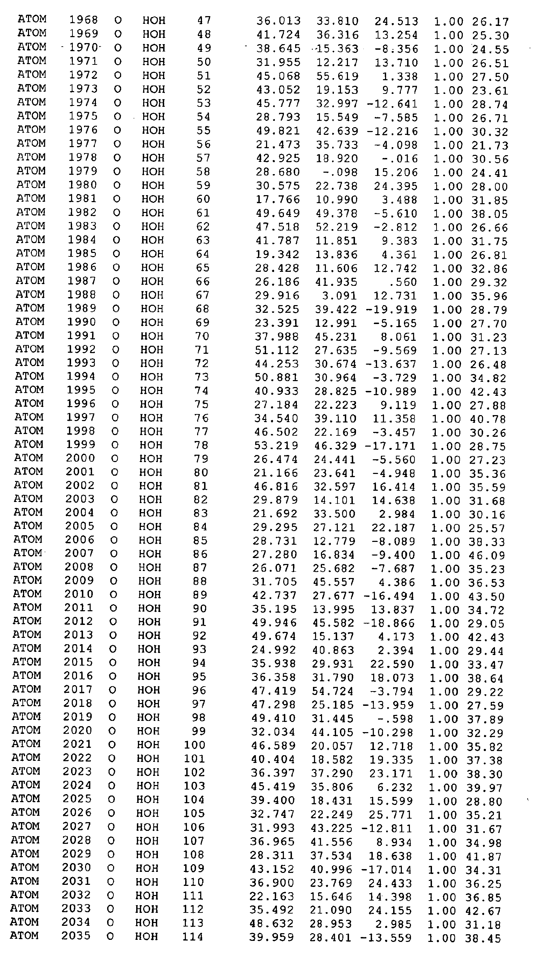

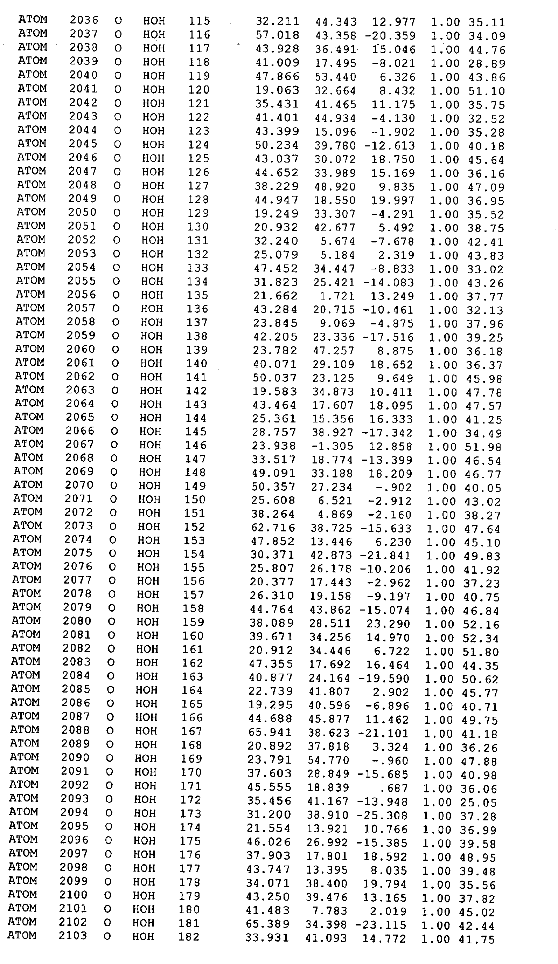

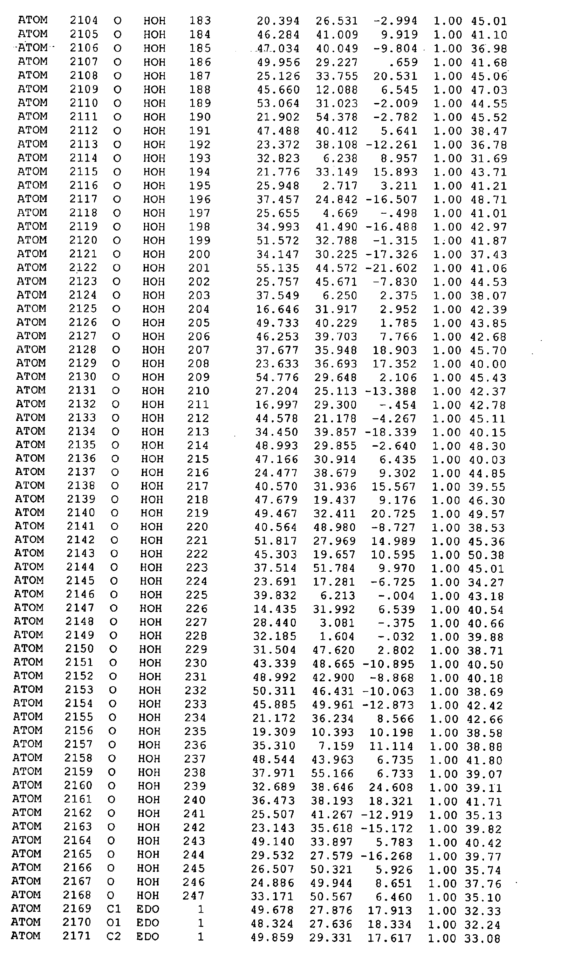

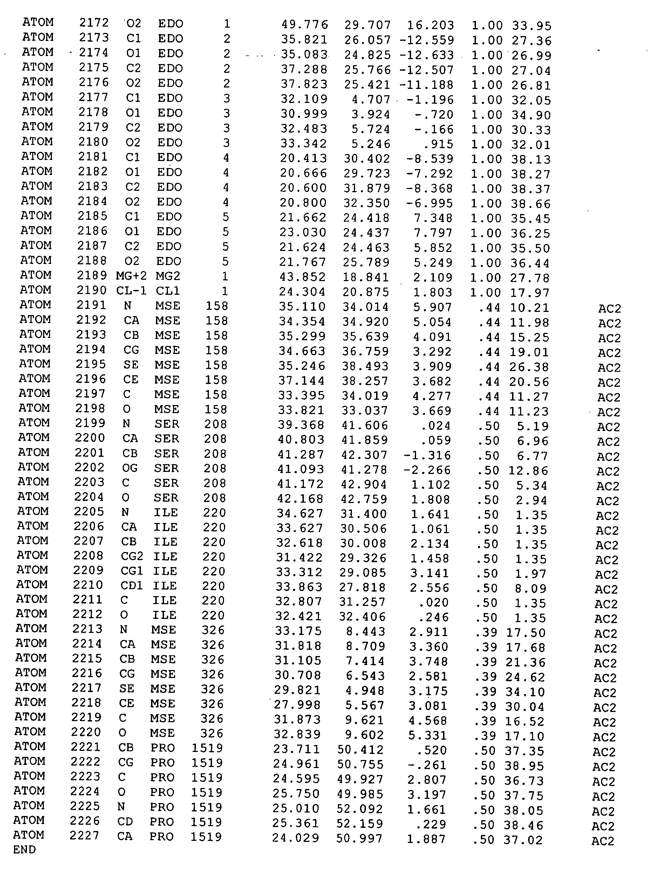

Figure 1 sets out Table 1, providing the coordinates of a RAD51-BRCA2 BRC4 complex structure,

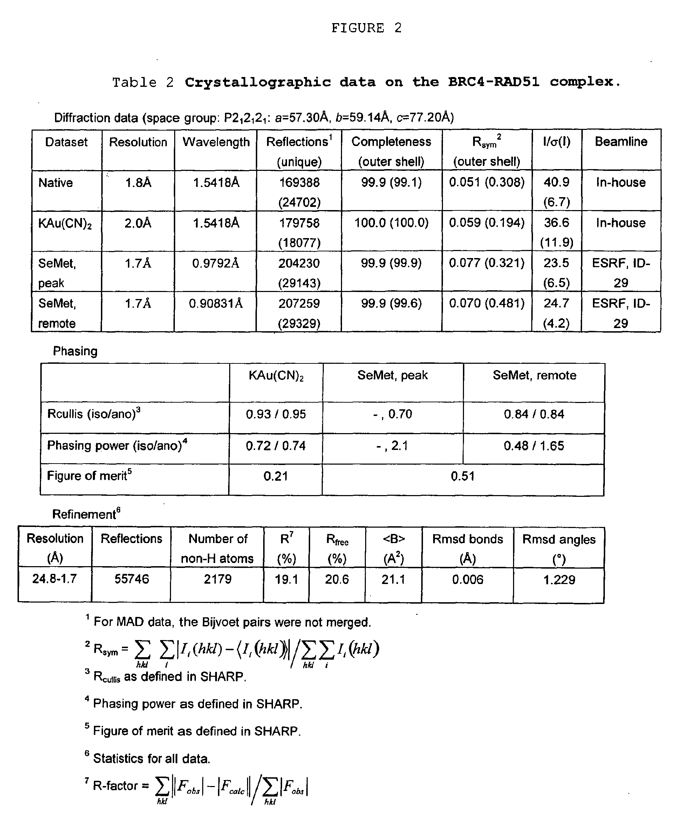

Figure 2 sets out Table 2, providing crystallographic data for the complex structure of Table 1,

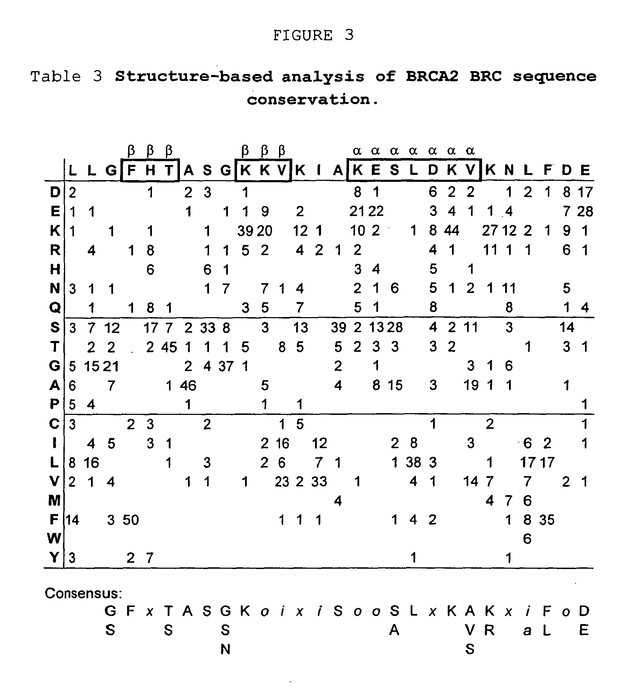

Figure 3 sets out Table 3, providing a structure-based analysis of BRCA2 BRC sequence conservation,

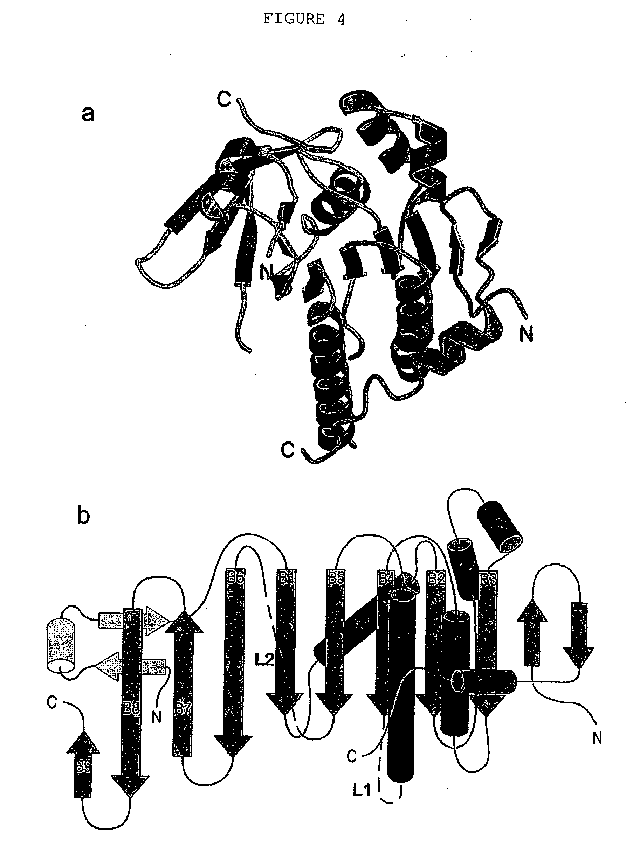

Figure 4 shows (a) ribbon representations of the RAD51 and BRC4 structures in the RAD51-BRCA2 BRC4 complex, the shorter BRC4 structure being positioned in front of the RAD51 structure and amino- and caboxyl-termini being indicated N and C respectively, and (b) a schematic topology diagram of the complex, the RAD51 secondary structures that are part of the RecA-homology domain being numbered and disordered RAD51 loops L1 and L2 respectively connecting beta strand B4 to alpha helix A5 and B5 to B6 shown as dashed lines (the flexible polypeptide linker connecting the RAD51 to BRC4 being omitted in both (a) and (b)),

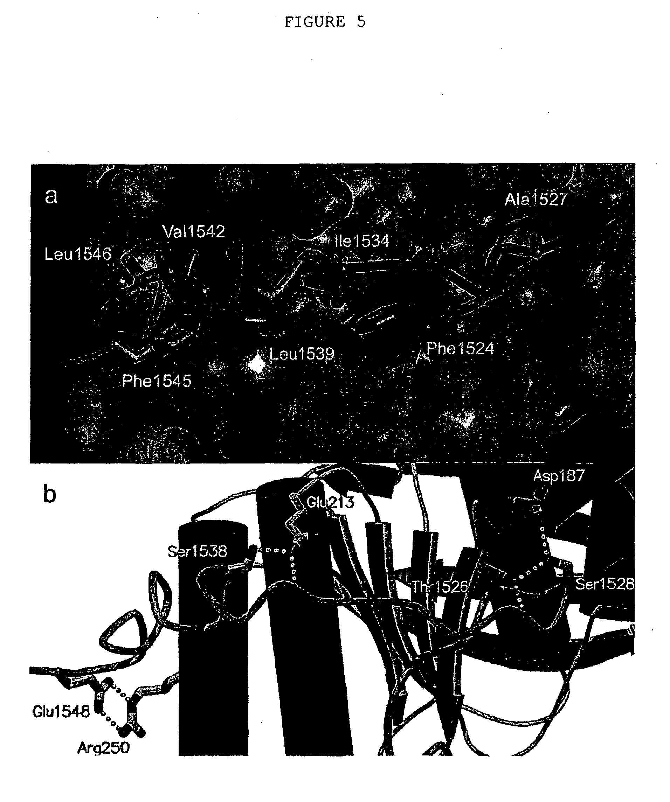

Figure 5 shows the interface of the RAD51-BRCA2 BRC4 complex as (a) a solvent-accessible molecular surface generated for the RAD51 interface residues and superimposed with a tube representing the BRC4 interface backbone chain, with stick representations of BRC4 side chains projecting from the BRC4 backbone chain, and (b) a ribbon diagram of the RAD51 interface residues superimposed with a tube representation of the BRC4 interface backbone chain, stick representations of BRC4 side chains projecting from the BRC4 backbone chain, RAD51 side chains projecting from the RAD51 ribbon diagram, and dashed lines representing hydrogen bonds,

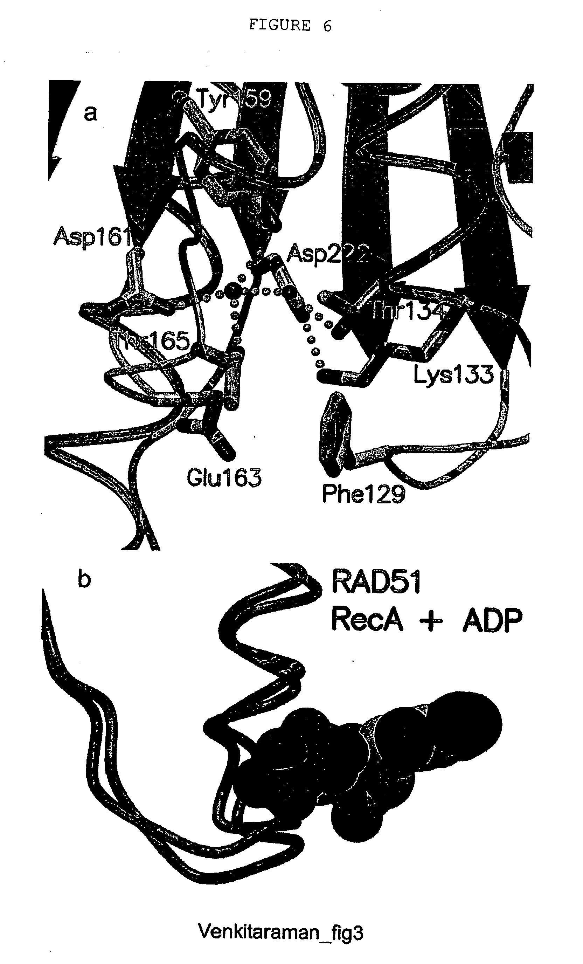

Figure 6 shows (a) a close view of the RAD51 ATP-binding pocket, side chains of residues important for ATP binding and hydrolysis, together with adjacent, interacting amino acids,' being shown as sticks, the sphere indicating the position of a buried water molecule, and dashed lines representing hydrogen bonds, and (b) a superposition of the phosphate-binding loops of RAD51 and ADP-bound RecA, the atoms of the ADP molecule being drawn as spheres of Van der Waals radii,

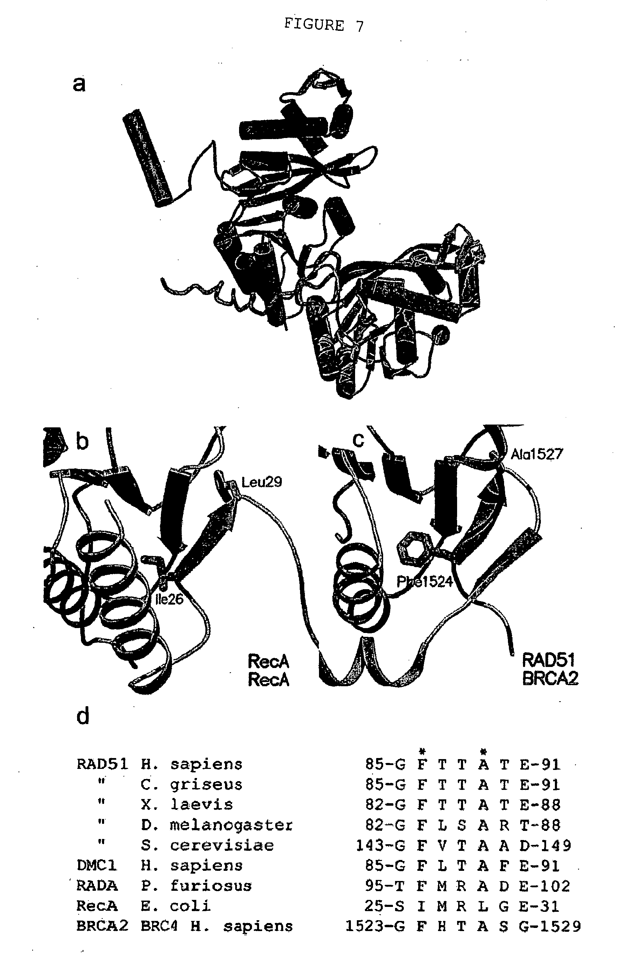

Figure 7 shows (a) a superposition of the RAD51-BRCA2 complex on a subunit of the crystallographic RecA filament (omitting RAD51 for clarity), the BRC motif being positioned at the interface between adjacent RecA subunits in the filament, (b) a close view of part of the interface between subunits in the crystallographic RecA filament, the sequence 26-IMRL-29 in the amino terminal tail of RecA mediating polymerisation by antiparallel beta strand pairing, and residues Ile26 and Leu29 representing points of hydrophobic contacts between subunits, (c) a close view of part of the interface between RAD51 and the BRC motif, the BRCA2 sequence 1524-FHTA-1527 interacting with RAD51 via antiparallel beta strand pairing, and residues Phe1524 and Ala1527 contacting RAD51 hydrophobically, and (d) a demonstration of evolutionary conservation of RAD51 residues predicted to be involved in nucleoprotein filament formation, sequences of human DMC1, pyrococcus (an archea bacterium) RADA, bacterial RecA and human BRCA2 with a comparable structural role being aligned underneath, and RAD51 residues completely or highly conserved being boxed, and

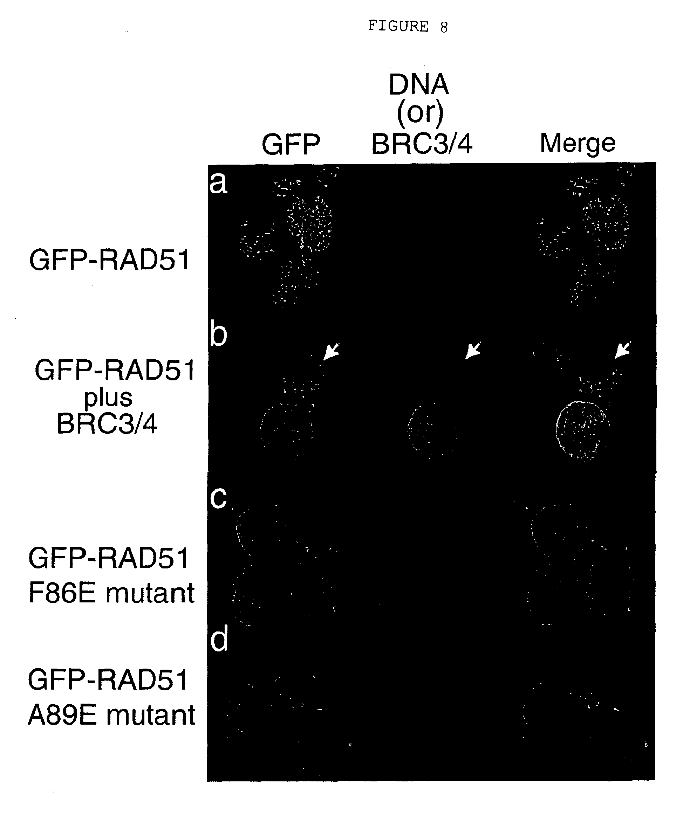

Figures 8(a) to (d) shows microscope images obtained from transfected 293T cells. Nuclei in the middle panels of (a), (c) and (d) are stained with the DNA dye ToPro3 (Molecular Probes). In (a) GFP-RAD51 accumulates in nuclear foci. In (b) focus formation is dependent on RAD51 multimerization because co-expression of BRC3/4 (middle panel) prevents GFP-RAD51 focus formation, resulting in its diffuse nuclear distribution. Merged staining in the right hand panel.marks cells that co-express GFP-RAD51 with BRC3/4. The cell denoted with a white arrow expresses GFP-RAD51 but not BRC3/4. GFP-RAD51 focus formation occurs in this cell, providing an internal experimental control. In (c) and (d) GFP-tagged mutants of RAD51 do not accumulate in foci.

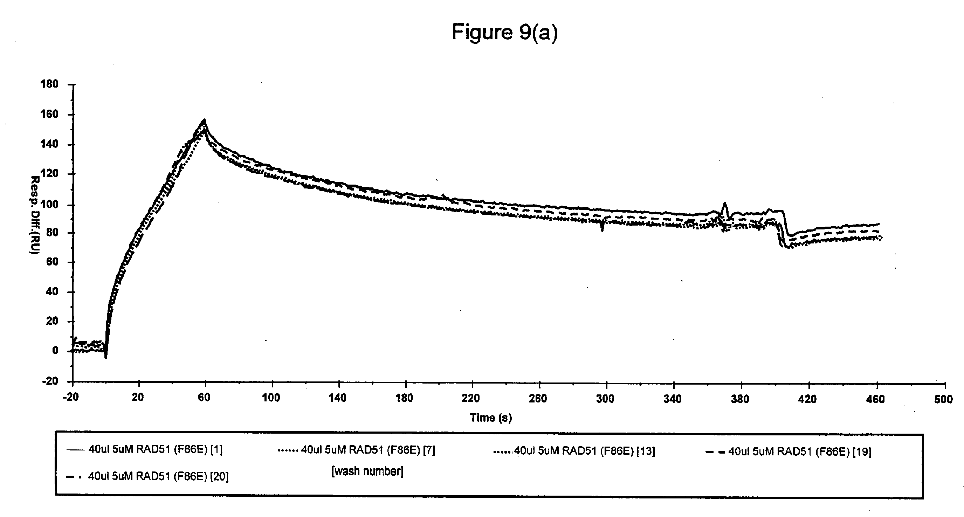

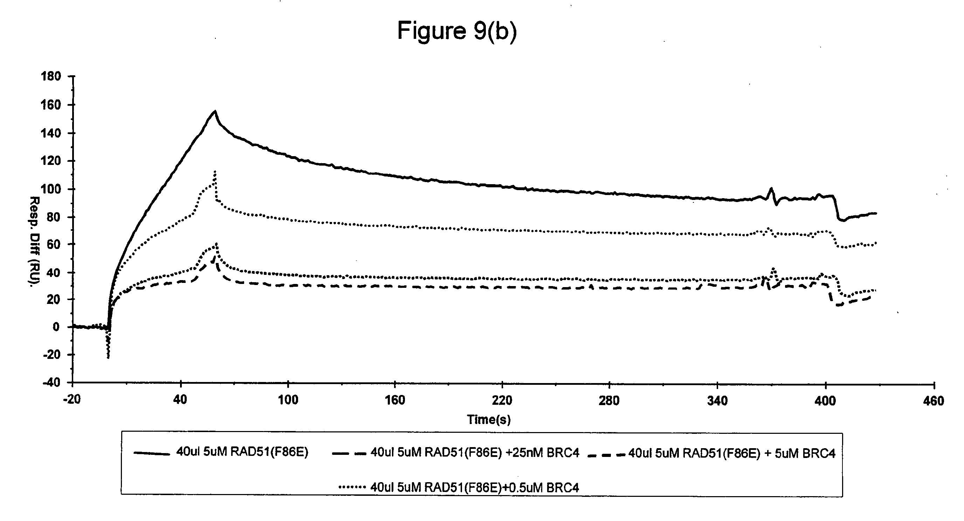

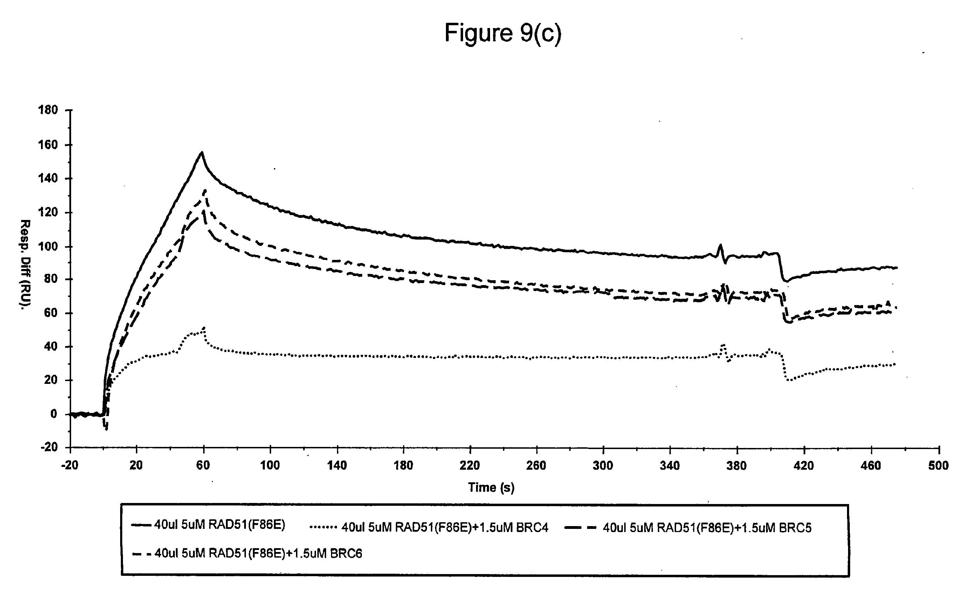

Figures 9(a) to (c) are sensorgrams showing the binding of 5 µM RAD51 F86E solution to BRC4 coupled-chips over time. In (a) the binding is repeated after a number of chip regenerations. (b) shows the binding of 5 µM RAD51 F86E solution, and solutions of 5 µM RAD51 F86E pre-incubated with non-biotinylated BRC4. (c) shows the binding of 5 µM RAD51 F86E solution, and solutions of 5 µM RAD51 F86E pre-incubated with different non-biotinylated BRC repeats.

### Detailed Description of the Invention

### A. Chimaeras

The present invention provides a RAD51-BRC repeat sequence chimaera protein in which the RAD51 is covalently joined to a BRC repeat sequence. The present invention further provides a nucleic acid encoding the chimaera protein.

Such a protein and such a nucleic acid may be obtained using the methods described in the accompanying examples.

By covalently binding RAD51 to a BRC repeat sequence we have formed a chimaera which for the first time allows RAD51 to be crystallized in a form suitable for X-ray structural analysis.

A flexible polypeptide linker (such as (Gly)₁₂, (Ser)₁₂, or (GlySer)₆) may be used to join the RAD51 and the BRC repeat sequence. Preferably the linker allows substantially unrestrained interaction between the BRC repeat sequence and the RAD51.

The RAD51 is preferably human RAD51. The RAD51 may be a wild-type protein or a variant thereof which is modified, for example by N-terminal truncation so that the truncated RAD51 spans the RecA homology domain. The BRC repeat sequence is preferably a BRCA2 BRC repeat, more preferably a human BRCA2 BRC repeat and even more preferably the human BRCA2 BRC3 or BRC4 repeat.

The same approach may be used to form chimaeras of RAD51 orthologues from other organisms, or RAD51 paralogues (such as DMC1, RAD51B, RAD51C, xrcc2, xrcc3, RAD52, RAD54, RAD55 and RAD57) with BRC repeat sequences or other peptides or polypeptides. The chimaeras should be crystallizable in a form suitable for X-ray structural analysis, even though, insofar as is known, the paralogues themselves have a tendency to agglomerate in solution like RAD51. Thus more general aspects of the present invention provide (a) a chimaera protein in which a RAD51 orthologue or paralogue is covalently joined to a BRCA2 BRC repeat, or other peptide or polypeptide and (b) a nucleic acid encoding the chimaera protein.

### B. Protein Crystals

In a further aspect, the present invention provides a crystal of a RAD51-BRC repeat sequence complex having the orthorhombic space group P2₁2₁2₁, and unit cell dimensions a = 57.30 Å, b = 59.14 Å, c = 77.20 Å. The crystal contains one complex in the asymmetric unit. Unit cell variability of 5% may be observed in all dimensions. The complex is preferably a RAD51-BRCA2 BRC repeat sequence complex.

Such a crystal may be obtained using the methods described in the accompanying examples. The RAD51 may be N-terminal truncated so that it spans the RecA homology domain. The RAD51-BRC repeat sequence complex may be formed by interaction between the RAD51 and BRC repeat sequence portions of a RAD51-BRC repeat sequence chimaera protein described above.

The methodology used to provide a RAD51-BRC repeat sequence complex crystal illustrated herein may be used generally to provide a RAD51-BRC repeat sequence complex crystal which diffracts X-rays for the determination of atomic coordinates of the complex to a resolution of better than 2.0 Å and preferably better than 1.8 or 1.7 Å.

The invention thus further provides a RAD51-BRC repeat sequence complex crystal which diffracts X-rays for the determination of atomic coordinates of the complex to a resolution of better than 2.0 Å and preferably better than 1.8 or 1.7 Å.

### C. Crystal Coordinates

In a further aspect, the present invention also provides a crystal of a RAD51-BRC repeat sequence complex having the three dimensional atomic coordinates of Table 1. An advantageous feature of the structure defined by the atomic coordinates is that it has a high resolution of about 1.7 Å.

Thus for the first time we have been able to provide atomic coordinate data for human RAD51 and a BRC repeat sequence of human BRCA2. More specifically we have provided atomic coordinate data for the interface between RAD51 and the BRC repeat sequence. As shown.in relation to the examples, these data reveal the structural basis for the BRCA2-dependent regulation of RAD51 function in DNA recombination, and provide insight into BRCA2 mutations associated with increased susceptibility to cancer.

Table 1 gives atomic coordinate data for a RAD51-BRC repeat sequence complex. In Table 1 the third column denotes the atom; the fourth the residue type; the fifth (where present) the chain identification (A is RAD51, B is BRC repeat sequence, C is an artificial tetrapeptide sequence, and AC1 and AC2 represent alternative side chain conformations for RAD51 amino acids 158, 208, 220, 326 and BRC repeat sequence amino acid 1519); the sixth the residue number (the residue numbering is with respect to the full length wild type protein); the seventh, eighth and ninth columns are the X, Y, Z coordinates respectively of the atom in question in Å; the tenth column the occupancy of the atom; the eleventh the temperature factor of the atom; and the twelfth (where present) the chain identification.

The coordinates of Table 1 provide a measure of atomic location in Å, to 3 decimal.places. The coordinates are a relative set of positions that define a shape in three dimensions, but.the skilled person would understand that an entirely different set of coordinates having a different origin and/or axes could define a similar or identical shape. Furthermore, the skilled person would understand that varying the relative atomic positions of the atoms of the structure so that the root mean square deviation of the residue backbone atoms (i.e. the nitrogen-carbon-carbon backbone atoms of the protein amino acid residues) is less than 2.0 Å, preferably less than 1.5 Å, more preferably less than 1.0 Å, even more preferably less than 0.64 Å and most preferably less than 0.5 Å, when superimposed on the coordinates provided in Table 1 for the residue backbone atoms, will generally result in a structure which is substantially the same as the structure of Table 1 in terms of both its structural characteristics and usefulness for RAD51/BRC repeat sequence structure-based analysis. Likewise the skilled person would understand that changing the number and/or positions of the water and ethylene glycol molecules and the magnesium and chloride ions of Table 1 will not generally affect the usefulness of the structure for structure-based analysis.

Thus for the purposes described herein as being aspects of the present invention, it is within the scope of the invention if: the Table 1 coordinates are transposed to a different origin and/or axes; the relative atomic positions of the atoms of the structure are varied so that the root mean square deviation of residue backbone atoms is less than 2.0 Å, preferably less than 1.5 Å, more preferably less than 1.0 Å, even more preferably less than 0.64 Å and most preferably less than 0.5 Å, when superimposed on the coordinates provided in Table 1 for the residue backbone atoms; and/or the number and/or positions of water molecules, ethylene glycol molecules, magnesium ions and/or chloride ions is varied.

Reference herein to the coordinate data of Table 1 thus includes the coordinate data in which one or more individual values of the Table are varied in this way. By "root mean square deviation" we mean the square root of the arithmetic mean of the squares of the deviations from the mean.

Those of skill in the art will appreciate that in many applications of the invention, it is not necessary to utilise all the coordinates of Table 1 but merely a portion of them. For example, as described below, in methods of modelling candidate compounds with RAD51 or BRC repeat sequences, selected coordinates from Table 1 may be used, for example at least 5, preferably at least 10, more preferably at least 50 and even more preferably at least 100 atoms of the RAD51-BRC repeat sequence structure. Likewise, the other applications of the invention described herein, including homology modelling and structure solution, and data storage and computer assisted manipulation of the coordinates, may also utilise all or a portion of the coordinates of Table 1.

### D. Mutants

A mutant is a protein characterized by replacement or deletion of at least one amino acid from the wild type protein, or insertion of at least one amino acid into the wild type protein. Such a mutant may be prepared for example by site- specific mutagenesis, or incorporation of natural or unnatural amino acids.

To produce mutants of RAD51 or BRCA2, amino acids present in RADS1 or BRCA2 can be replaced by other amino acids having similar or contrary properties, for example hydrophobicity, hydrophobic moment, antigenicity, propensity to form or break α-helical or β-sheet structures, and so on. Substitutional variants of a protein are those in which at least one amino acid in the protein sequence has been removed and a different residue inserted in its place. Amino acid substitutions are typically of single residues but may be clustered depending on functional constraints e.g. at a crystal contact. Insertional, amino acid variants are those in which one or more amino acids are introduced. This can be amino-terminal and/or carboxy-terminal fusion as well as intrasequence. Examples of amino-terminal and/or carboxy-terminal fusions are affinity tags, MBP tags, and epitope tags.

In some instances, it may be particularly advantageous or convenient to substitute, delete and/or add amino acid residues to a RAD51 or BRCA2 binding pocket or catalytic residue in order to provide convenient cloning sites in cDNA encoding the polypeptide, to aid in purification of the polypeptide, etc. Such substitutions, deletions and/or additions which do not substantially alter the three dimensional structure of RAD51 or the BRCA2 will be apparent to those having skills in the art.

It should be noted that the mutants contemplated herein need not exhibit enzymatic activity. Indeed, amino acid substitutions, additions or deletions that interfere with the activity of RAD51 or BRCA2 but which do not significantly alter the three-dimensional structure of the catalytic region are specifically contemplated by the invention. Such crystalline polypeptides, or the atomic structure co-ordinates obtained there from, can be used to identify compounds that bind to the protein.

One aspect of the present invention.provides a mutant RAD51 which has been modified to reduce or eliminate the tendency of RAD51 to spontaneously aggregate into high molecular weight complexes. Thus preferably the mutant RAD51 maintains a monomeric form in solution. The present invention further provides a nucleic acid encoding the mutant RAD51.

The formation of such mutants is described in the accompanying examples. The mutant may be formed by substitution, deletion and/or addition of at least one amino acid in the 85-GFTTATE-91 sequence of human RAD51, or the corresponding sequence in other forms of RAD51.

Such corresponding sequences in other forms of RAD51 are highly conserved and are readily identifiable e.g. by sequence alignment techniques. The sequences for mouse, hamster, fruit fly and yeast are provided in the accompanying examples.

Preferably the mutation substantially alters the functionality of the sequence. For example, in the accompanying examples we replaced the hydrophobic residue Phe86 or Ala89 in the 85-GFTTATE-91 sequence of human RAD51 with hydrophilic glutamic acid. Other suitable mutations would be apparent to the skilled person.

Advantageously, the mutant RAD51 may be crystallised in a form suitable for further X-ray analysis of the RAD51 structure. The mutant RAD51 may also be used in an assay for identifying compounds (e.g. proteins) which interact with or form part of a RAD51 pathway.

### E. Homology Modelling

The invention also provides a means for homology modelling of other proteins (referred to below as target proteins). By "homology modelling", it is meant the prediction of related RAD51 or BRC repeat sequence structures based either on X-ray crystallographic data or computer-assisted de novo prediction of structure, and involving the manipulation of the coordinate data of Table 1.

Homology modelling as such is a technique that is well known to those skilled in the art (see e.g. Greer, Science, Vol. 228, (1985), 1055, and Blundell *et al., Eur. J. Biochem,* Vol. 172, (1988), 513). The techniques described in these references, as well as other homology modelling techniques generally available in the art, may be used in performing the present invention.

Homology modelling extends to target proteins which are analogues or homologues of the RAD51 or BRC repeat sequence whose structures have been determined in the accompanying examples. It also extends to protein mutants of the RAD51 or BRC repeat sequence.

In general, the method involves comparing the amino acid sequences of the RAD51 or BRC repeat of Table 1 with a target protein by aligning the amino acid sequences. Amino acids in the sequences are then compared and groups of amino acids that are homologous (conveniently referred to as "corresponding regions") are grouped together. This method detects conserved regions of the polypeptides and accounts for amino acid insertions or deletions.

Homology between amino acid sequences can be determined using commercially available algorithms. The programs BLAST, *gapped BLAST, BLASTN,* PSI-BLAST and *BLAST* 2 sequences (provided.by the National Center for Biotechnology Information) are widely used in the art for this purpose, and can align homologous regions of two amino acid sequences. These may be used with default parameters to determine the degree of homology between the amino acid sequence from Table 1 and other target proteins which are to be modelled.

Analogues are defined as proteins with similar three-dimensional structures and/or functions and little evidence of a common ancestor at a sequence level.

Homologues are defined as proteins with evidence of a common ancestor i.e. likely to be the result of evolutionary divergence and are divided into remote, medium and close subdivisions based on the degree (usually expressed as a percentage) of sequence identity.

A homologue is defined here as a protein which has at least 15% sequence identity with RAD51 in the RecA homology domain or with a BRC repeat sequence, or one functional domain which is characteristic of RAD51 in the RecA homology domain or of a BRC repeat sequence.

There are two types of homologue: orthologues and paralogues. Orthologues are defined as homologous genes in different organisms, i.e. the genes share a common ancestor coincident with the speciation event that generated them. Paralogues are defined as homologous genes in the same organism derived from a gene/chromosome/genome duplication, i.e. the common ancestor of the genes occurred since the last speciation event.

For the purpose of homology modelling, the present invention also contemplates mutants which are polypeptides obtained (a) by replacing at least one amino acid residue in the native or synthetic RecA homology domain of RAD51 with a different amino acid residue and/or (b) by adding and/or deleting at least one amino acid residue within and/or at the N- and/or C-terminus of the native or synthetic RecA homology domain of RAD51, the polypeptide corresponding to the RecA homology domain of RAD51 and having substantially the same three-dimensional structure as the RecA homology domain of RAD51 from which it is derived.

For the purpose of homology modelling, the present invention further contemplates mutants which are polypeptides obtained (a) by replacing at least one amino acid residue in a native or synthetic BRC repeat sequence with a different amino acid residue and/or (b) by adding and/or deleting at least one amino acid residue within and/or at either or both ends of a native or synthetic BRC repeat sequence, the polypeptide having one or more sequences corresponding to a BRC repeat sequence and in those sequences having substantially the same three-dimensional structure as the BRC repeat from which they are derived.

By having substantially the same three-dimensional structure is meant having a set of atomic structure co-ordinates that have a root mean square deviation (r.m.s.d.) of less than or equal to about 2.0 Å when superimposed with the atomic structure co-ordinates of the RAD51 from which the mutant is derived when at least about 50% to 100% of the C_{α} atoms of the RAD51 are included in the superposition.

Once the amino acid sequences of the polypeptides with known and unknown structures are aligned, the structures of the conserved amino acids in a computer representation of the polypeptide with known structure are transferred to the corresponding amino acids of the polypeptide whose structure' is unknown. For example, a tyrosine in the amino acid sequence of known structure may be replaced by a phenylalanine, the corresponding homologous amino acid in the amino acid sequence of unknown structure.

The structures of amino acids located in non-conserved regions may be assigned manually by using standard peptide geometries or by molecular simulation techniques, such as molecular dynamics. The final step in the process is accomplished by refining the entire structure using molecular dynamics and/or energy minimization.

Thus the invention provides a method of homology modelling comprising the steps of:
(a) aligning a representation of an amino acid sequence of a target protein of unknown three-dimensional structure with the amino acid sequence of the RAD51 or the BRC repeat sequence of Table 1 to match homologous regions of the amino acid sequences;
(b) modelling the structure of the matched homologous regions of said target protein of unknown structure on the corresponding regions of the RAD51 or BRC repeat sequence structure as defined by Table 1; and
(c) determining a conformation (e.g. so that favourable interactions are formed within the target protein of unknown structure and/or so that a low energy conformation is formed) for said target protein of unknown structure which substantially preserves the structure of said matched homologous regions.

Preferably one or all of steps (a) to (c) are performed by computer modelling.

In respect of RAD51, the data of Table 1 will be particularly advantageous for homology modelling of proteins such as DMC1, RAD51B, RAD51C, xrcc2, xrcc3, RAD52, RAD54, RAD55 andRAD57. These proteins may be the target protein in the method of the invention described above.

### F. Structure Solution

The structure of the RAD51-BRC repeat sequence complex can also be used to solve the crystal structure of other target proteins such as other crystal forms of RAD51, RAD51 mutants, RAD51 homologues, and other complexes of RAD51, and corresponding crystal forms relating to a BRC repeat sequence, where X-ray diffraction data of these target proteins has been generated and requires interpretation in order to provide a structure.

Thus, where X-ray crystallographic or NMR spectroscopic data is provided for a target protein of unknown three-dimensional structure, the structure of the RAD51-BRC repeat sequence complex as defined by Table 1 may be used to interpret that data to provide a likely structure for the target protein by techniques which are well known in the art, e.g. phasing in the case of X-ray crystallography and assisting peak assignments in NMR spectra.

One method that may be employed for these purposes is molecular replacement. In this method, the unknown crystal structure may be determined using the RAD51 or BRC repeat sequence structure coordinates of this invention as provided herein. This method will provide an accurate structural form for the unknown crystal more quickly and efficiently than attempting to determine such information ab initio.

Examples of computer programs known in the art for performing molecular replacement are CNS (Brunger A.T.; Adams P.D.; Rice L.M., Current Opinion in Structural Biology, Volume 8, Issue 5, October 1998, Pages 606-611 (also commercially available from Accelerys San Diego, CA) or AMORE (Navaza, J. (1994). AMoRe: an automated package for molecular replacement. Acta Cryst. A50, 157-163).

Thus, in a further aspect the invention provides a method for determining the structure of a protein, which method comprises;
providing the co-ordinates of Table 1, and
positioning the co-ordinates in the crystal unit cell of said protein so as to provide a structure for said protein.

In a preferred aspect of this invention the RAD51 co-ordinates are used to solve the structure of, for example, DMC1, RAD51B, RAD51C, xrcc2, xrcc3, RAD52, RAD54, RAD55 or RAD57.

The invention may also be used to assign peaks of NMR spectra of such proteins, by manipulation of the data of Table 1.

### G. Computer Systems

In another aspect, the present invention provides a system, particularly a computer system, the system containing either:
(a) atomic coordinate data according to Table 1, said data defining the three-dimensional structure of the RAD51-BRC repeat sequence complex or at least selected coordinates thereof;
(b) structure factor data (where a structure factor comprises the amplitude and phase of the diffracted wave) for the RAD51-BRC repeat sequence complex, said structure factor data being derivable from the atomic coordinate data of Table 1;
(c) atomic coordinate data of a target protein generated by homology modelling of the target based on the data of Table 1;
(d) atomic coordinate data of a target protein generated by interpreting X-ray crystallographic data or NMR data by reference to the data of Table 1; or
(e) structure factor data derivable from the atomic coordinate data of (c) or (d) .

Such data is useful for a number of purposes, including the generation of structures to analyse the mechanisms of action of RAD51, BRC repeat sequences or related proteins and/or to perform rational drug design of compounds which interact with RA51 or BRC repeat sequences.

As used herein, "a computer system" refers to the hardware means, software means and data storage means used to analyse the atomic coordinate and/or structure factor data of the present invention. The minimum hardware means of the computer-based systems of the present invention typically comprises a central processing unit (CPU), a working memory and data storage means, and'e.g. input means, output means etc. Desirably a monitor is provided to visualize structure data The data storage means may be RAM or means for accessing a computer readable medium of the invention. Examples of such systems are microcomputer workstations available from Silicon Graphics Incorporated and Sun Microsystems running Unix based, Windows NT or IBM OS/2 operating systems.

In a further aspect, the present invention provides a computer readable storage medium on which is stored thereon either:
(a) atomic coordinate data according to Table 1, said data defining the three-dimensional structure of the RAD51-BRC repeat sequence complex or at least selected coordinates thereof;
(b) structure factor data (where a structure factor comprises the amplitude and phase of the diffracted wave) for the RAD51-BRC repeat sequence complex, said structure factor data being derivable from the atomic coordinate data of Table 1;
(c) atomic coordinate data of a target protein generated by homology modelling of the target based on the data of Table 1;
(d) atomic coordinate data of a target protein generated by interpreting X-ray crystallographic data or NMR data by reference to the data of Table 1; or
(e) structure factor data derivable from the atomic coordinate data of (c) or (d).

As used herein, "computer-readable storage medium" refers to any medium or media which can be read and accessed directly by a computer. Such media include, but are not limited to: magnetic storage media such as floppy discs, hard disc storage medium and magnetic tape; optical storage media such as optical discs or CD-ROM; electrical storage media such as RAM and ROM; and hybrids of these categories such as magnetic/optical storage media.

By providing such a storage medium, the atomic coordinate data can be routinely accessed to model RAD51, a BRC repeat sequence, or selected coordinates thereof. For example, RASMOL (Sayle et al., *TIBS,* Vol. 20, (1995), 374) is a publicly available computer software package which allows access and analysis of atomic coordinate data for structure determination and/or rational drug design.

On the other hand, structure factor data, which are derivable from atomic coordinate data (see e.g. Blundell et al., in *Protein Crystallography,* Academic Press, New York, London and San Francisco, (1976)), are particularly useful for calculating e.g. difference Fourier electron density maps.

A further aspect of the invention provides a method of providing data for generating structures and/or performing drug design with RAD51/BRC repeat sequences, RAD51/BRC repeat sequence homologues or analogues, complexes of RAD51/BRC repeat sequence with a compound, or complexes of RAD51/BRC repeat sequence homologues or analogues with compounds, the method comprising:
(i) establishing communication with a remote device containing computer-readable data comprising at least one of: (a) atomic coordinate data according to Table 1, said data defining the three-dimensional structure of the RAD51-BRC repeat sequence complex or at least selected coordinates thereof; (b) structure factor data (where a structure factor comprises the amplitude and phase of the diffracted wave) for the RAD51-BRC repeat sequence complex, said structure factor data being derivable from the atomic coordinate data of Table 1; (c) atomic coordinate data of a target protein generated by homology modelling of the target based on the data of Table 1; (d) atomic coordinate data of a target protein generated by interpreting X-ray crystallographic data or NMR data by reference to the data of Table 1; or (e) structure factor data derivable from the atomic coordinate data of (c) or (d); and
(ii) receiving said computer-readable data from said remote device.

Thus the remote device may comprise e.g. a computer system or a computer-readable storage medium of one of the previous aspects of the invention.'The device may be in a different country or jurisdiction from where the computer-readable data is received.

The communication may be via the internet, intranet, e-mail etc. Typically the communication will be electronic in nature, but some or all of the communication pathway may be optical, for example, over optical fibers.

### H. Uses of the Structure of the Invention

The crystal structure obtained according to the present invention may be used in several ways for drug design.

We show in the examples below that the BRC repeats encoded in BRCA2 structurally mimic a sequence in RecA that contributes to the interface between successive subunits in the RecA filament, and we present evidence that RAD51 multimerization in nucleoprotein filament formation proceeds through a similar interface. The sequence 85-GFTTATE-91 in RAD51 closely resembles the conserved BRC repeat sequence (GFxTASG) that mimics RecA. Furthermore, replacement of Phe86 or Ala89 in RAD51 with glutamic acid, predicted to disrupt critical hydrophobic contacts, creates mutants that are no longer capable of filament formation when expressed in mammalian cells. Thus, our findings uncover an evolutionarily conserved structural motif that enables RecA and RAD51 to assemble into multimeric filaments essential for DNA recombination, and that has become incorporated into BRCA2, a protein exclusive to higher eukaryotes.

Our work provides a structural rationale for the conservation of residues in different BRC repeats from several different species. Alteration of certain of these residues by cancer-associated mutations is predicted to perturb RAD51 binding, emphasizing the importance of the RAD51-BRC repeat interaction as a target for BRCA2 mutations associated with cancer susceptibility.

BRC repeats are found not only in BRCA2 of vertebrates but also in novel proteins of uncertain function expressed in several parasitic species (such as Leishmania and trypanosomes), which our structural analysis suggests will bind and regulate RAD51 orthologues expressed in those species in a manner similar to BRC4. Thus the RAD51-BRC repeat structure may have a role in identifying compounds for treating parasite infection.

Structure-based analysis also identifies several residues in BRC repeats and in RAD51 whose modification by phosphorylation or other means is predicted to affect complex formation, providing a means of linking BRCA2-RAD51 regulation to the pathways that signal DNA damage, blocked replication or cell cycle progression.

Thus our findings provide a structural blueprint that may be useful in structure based drug design. Our work shows that the RAD51-BRCA2 interaction will be particularly vulnerable to small molecule inhibitors because it critically depends on spatially constrained hydrophobic contacts to RAD51 made by three residues (Phe1526, Phe1546 and Ala1527) in BRC4, also conserved in different BRC repeats. Because BRCA2 and RAD51 participate in the repair of DNA breakage ^{8,9}, such inhibitors may prove useful adjuncts to radiation therapy or anti-cancer drugs that induce DNA damage or block DNA replication.

Therefore, the determination of the three-dimensional structure of the RAD51-BRC repeat sequence complex provides a basis for the design of new compounds which interact with RAD51 and/or BRC repeat sequences in novel ways.

### H,1. Obtaining and Analysing Crystal Complexes

In one approach, the structure of a compound bound to RAD51 or a BRC repeat sequence may be determined by experiment. This will provide a starting point in the analysis of the compound bound to RAD51 or the BRC repeat, thus providing those of skill in the art with a detailed insight as to how that particular compound interacts with RAD51 or a BRC repeat sequence.

Many of the techniques and approaches to structure-based drug design described rely at some stage on X-ray analysis to identify the binding position of a ligand in a ligand-protein complex. A common way of doing this is to perform X-ray crystallography on the complex, produce a difference Fourier electron density map, and associate a particular pattern of electron density with the ligand. However, in order to produce the map (as explained e.g. by Blundell et al., mentioned above) it is necessary to know beforehand the protein 3D structure (or at least the protein structure factors). Therefore, determination of the BRCA2 BRC repeat sequence and RAD51 structures also allows production of difference Fourier electron density maps of RAD51- or BRC repeat sequence-compound complexes and determination of the binding position of a drug, and hence may greatly assist the process of rational drug design.

Accordingly, the invention provides a method for determining the structure of a compound bound to RAD51 or a BRC repeat sequence, said method comprising:
providing a crystal of a complex in which the compound is bound to RAD51 or a BRC repeat sequence; and
determining the structure of said complex by employing the data of Table 1.

The analysis of such structures may employ (i) X-ray crystallographic diffraction data from the complex and (ii) a three-dimensional structure of RAD51 or the BRC repeat sequence, or at least selected coordinates thereof, to generate a difference Fourier electron density map of the complex, the three-dimensional structure being defined by atomic coordinate data according to Table 1. The difference Fourier electron density map may then be analysed.

Therefore, such complexes can be crystallized and analysed using X-ray diffraction methods, e.g. according to the approach described by Greer et al., *J. of Medicinal Chemistry,* Vol. 37, (1994), 1035-1054, and difference Fourier electron density maps can be calculated based on X-ray diffraction patterns of complexes containing RAD51 or the BRC repeat sequence and the solved structure of RAD51 or the BRC repeat sequence according to Table 1. These maps can then be analysed e.g. to determine whether and where a particular compound binds to RAD51 or the BRC repeat sequence and/or changes the conformation of RAD51 or the BRC repeat sequence.

Electron density maps can be calculated using programs such as those from the CCP4 computing package (Collaborative Computational Project 4. The CCP4 Suite: Programs for Protein Crystallography, *Acta Crystallographica,* D50, (1994), 760- 763.). For map visualization and model building programs such as "0" (Jones et al., *Acta Crystallograhica,* A47, (1991), 110-119) can be used.

In addition, in accordance with this invention, RAD51 or BRC repeat sequence mutants may be crystallized in co-complex with known RAD51 or BRC repeat sequence substrates, inhibitors or novel compounds. The crystal structures of a series of such complexes may then be solved by molecular replacement and compared with that of the structure of Table 1. Potential sites for modification within the various binding sites of the mutant may thus be identified. This information provides an additional tool for determining the most efficient binding interactions, for example, increased hydrophobic interactions, between RAD51 and a chemical entity or compound.

### H.2. In Silico Analysis and Design

Although the invention will facilitate the determination of actual crystal structures comprising RAD51 or a BRC repeat sequence and a compound which interacts with RAD51 or the sequence repeat, current computational techniques provide a powerful alternative to the need to generate such crystals and generate and analyse diffraction data. Accordingly, a particularly preferred aspect of the invention relates to in *silico* methods directed to the analysis and development of compounds which interact with the RAD51 structure or the BRC repeat sequence structure of the present invention.

Thus as a result of the determination of the RAD51-BRC repeat sequence complex three-dimensional structure, more purely computational techniques for rational drug design may also be used to design structures whose interaction with RAD51 or the BRC repeat sequence is better understood. (for an overview of these techniques see e.g. Walters et al *(Drug Discovery Today,* Vol.3, No.4, (1998), 160-178). For example, automated ligand- receptor docking programs (discussed e.g. by Jones et al. in *Current Opinion in Biotechnology,* Vol.6, (1995), 652-656) which require accurate information on the atomic coordinates of target receptors may be used.

The aspects of the invention described herein which utilize the RAD51 or the BRC repeat sequence structure *in silico* may be equally applied to both the structure of Table 1 and the models of target proteins obtained by other aspects of the invention. Thus having determined a conformation of a target protein by the method described above, such a conformation may be used in a computer-based method of rational drug design as described herein.

Accordingly, the invention provides a computer-based method for the analysis of the interaction of a molecular structure with a RAD51 or BRC repeat sequence structure of the invention, which comprises:
providing the structure of a RAD51 or BRC repeat sequence of the invention;
providing a molecular structure to be fitted to said RAD51 or BRC repeat sequence structure; and
fitting the molecular structure to the RAD51 or BRC repeat sequence structure.

In an alternative aspect, the method of the invention may utilize the coordinates of atoms of interest of the RAD51 or BRC repeat sequence which are in the vicinity of a putative molecular structure binding region in order to model the pocket in which the structure binds. These coordinates may be used to define a space which is then analysed in silico. Thus the invention provides a computer-based method for the analysis of molecular structures which comprises:
providing the coordinates of at least two atoms of a RAD51 or BRC repeat sequence structure of the invention ("selected coordinates");
providing a molecular structure to be fitted to said coordinates; and
fitting the structure to the selected coordinates of the RAD51 or BRC repeat sequence.

In practice, it will be desirable to model a sufficient number of atoms of the RAD51 or BRC repeat sequence as defined by the coordinates of Table 1 which represent a binding region. Thus, in this embodiment of the invention, there will preferably be provided the coordinates of at least 5, preferably at least 10, more preferably at least 50 and even more preferably at least 100 selected atoms of the RAD51 or BRC repeat sequence structure.

Preferably the selected atoms are atoms which are identified below as contributing to interactions in the RAD51-BRC4 interface or being involved in the RAD51 nucleotide-binding site.

Although different compounds may interact with different parts of the binding region of the RAD51 or BRC repeat sequence, the structure of the RAD51 or BRC repeat sequence allows the identification of a number of particular sites which are likely to be involved in many of the interactions of RAD51 or a BRC repeat sequence with the compound (which may be e.g. a drug candidate). The residues are set out in the accompanying example. Thus in this aspect of the invention, the selected coordinates may comprise coordinates of some or all of these residues.

In order to provide a three-dimensional structure of compounds to be fitted to a RAD51 or BRC repeat sequence structure of the invention, the compound structure may be modeled in three dimensions using commercially available software for this purpose or, if its crystal structure is available, the coordinates of the structure may be used to provide a representation of the compound for fitting to a RAD51 or BRC repeat sequence structure of the invention.

By "fitting", it is meant determining by automatic, or semi-automatic means, interactions between at least one atom of a molecular structure and at least one atom of a RAD51 or BRC repeat sequence structure of the invention, and calculating the extent to which such an interaction is stable. Interactions include attraction and repulsion, brought about by charge, steric considerations and the like. Various computer-based methods for fitting are described further herein.

More specifically, the interaction of a compound with a RAD51 or BRC repeat sequence can be examined through the use of computer modelling using a docking program such as GRAM, DOCK, or AUTODOCK (see Walters et al., *Drug Discovery Today,* Vol.3, No.4, (1998), 160-178, and Dunbrack et al., *Folding and Design,* 2, (1997), 27-92). This procedure can include computer fitting of compounds to the RAD51 or BRC repeat sequence to ascertain how well the shape and the chemical structure of the compound will bind to the RAD51 or BRC repeat sequence.

Also computer-assisted, manual examination of the binding region structure of RAD51 or a BRC repeat sequence may be performed. The use of programs such as GRID (Goodford, J. *Med. Chem.,* 28, (1985), 849-857) - a program that determines probable interaction sites between molecules with various functional groups and an enzyme surface - may also be used to analyse the active site to predict, for example, the types of modifications which will alter binding interactions with a compound.

Detailed structural information can thus be obtained about the binding of the compound to RAD51 or a BRC repeat sequence, and in the light of this information adjustments can be made to the structure or functionality of the compound, e.g. to alter its interaction with RAD51 or the BRC repeat sequence. The above steps may be repeated and re-repeated as necessary.

Since the BRC repeat sequence is a natural ligand and inhibitor of RAD51, structural and spatial information can be usefully derived from the 3D structure of the RADS1-BRC repeat sequence complex, to facilitate the identification of a compound that interacts with RAD51 by partially or completely mimicking the mode of interaction found in the complex. A pharmacophore, or more specifically a spatial arrangement of a small group of atoms or a functional group, with a positive contribution to compound affinity toward RAD51, can be derived by an analysis of the geometry of the RAD51-BRC repeat sequence interface. Such a pharmacophore-based approach can be applied in drug discovery. An aspect of the invention thus relates to the use of the RAD51 structure or the BRC repeat sequence structure, or information derived from them, for the design or identification of a compound that mimics the BRC repeat sequence in its mode of interaction with RAD51.

One application is the identification of a compound that satisfies a specified pharmacophore. Accordingly, the invention provides a method for the analysis of molecular structures which comprises:
providing the coordinates of at least two atoms of a RAD51 or BRC repeat sequence structure of the invention;
assigning chemical properties to a spatial arrangement derived from the coordinates; and
providing a molecular structure that satisfies the chemical properties in the specified spatial arrangement.

In one application, the specified pharmacophore can be used for scoring compounds fitted against RAD51, an aim being to select compounds that fulfil the criteria of the pharmacophore, or to screen out, from a number of compounds, those that do not fulfil the criteria. Thus, the method may further comprise:
fitting the structure to the selected coordinates; and
evaluating the fitting based on the extent to which the chemical properties of the specified spatial arrangement are satisfied.

In general, the present invention provides for the use of the structure of a RAD51 or BRC repeat sequence of the invention, or for the use of selected coordinates of the structure, for analysing, designing or screening candidate compounds which (a) share RAD51 or BRC repeat sequence activity, (b) interact with RAD51 or BRC repeat sequence, (c) inhibit RAD51 multimerisation, or (d) inhibit or promote RAD51-BRC binding.

### H.3. compounds of the Invention.

Where the molecular structure of a compound which fits to the RAD51 or the BRC repeat sequence structure of the invention has been identified, the invention further includes the step of obtaining or synthesizing the compound and testing it in an in *vivo* or in *vitro* biological system in order to determine its activity (e.g. its ability to interact with RAD51 or to inhibit RAD51 multimerisation).

For example, compounds that fulfil the criteria of a specified pharmacophore can be assayed for activity against RAD51. Thus the invention may further comprise:
obtaining or synthesizing a compound having a molecular structure which satisfies the pharmacophore, and assaying the compound *in vivo* or *in vitro* in order to determine its activity.

In another aspect, the invention includes a compound which is identified by the methods of the invention described above.

Following identification of such a compound, it may be manufactured and/or used in the preparation, i.e. manufacture' or formulation, of a composition such as a medicament, pharmaceutical composition or drug. These may be administered to individuals.

Thus, the present invention extends in various aspects not only to a compound as provided by the invention, but also a pharmaceutical composition, medicament, drug or other composition comprising such a compound e.g. for treatment (which may include preventative treatment) of disease;'a method comprising administration of such a composition to a patient, e.g. for treatment of disease; .use of such an inhibitor in the manufacture of a composition for administration, e.g. for treatment of.disease; and a method of making a pharmaceutical composition comprising admixing such an inhibitor with a pharmaceutically acceptable excipient, vehicle or carrier, and optionally other ingredients.

The invention is illustrated by the following examples and analysis:

### I. Examples and Analysis

### I.1. Protein Expression and Purification

In order to favour BRCA2 binding over RAD51 multimerisation, we covalently joined the BRC repeat to RAD51. The BRCA2 BRC type 4 sequence (amino acids 1517 to 1551) was connected to the amino terminus of a RAD51 sequence spanning the RecA homology domain (Ser97 to the natural carboxyl terminus) via the flexible polypeptide linker: (ThrGlySer)₄MetGly, designed to allow for unrestrained interaction between the BRC repeat sequence and RAD51. The chimaeric protein was expressed in *E. coli* fused to a double amino-terminal tag consisting of a six histidine sequence followed by a GST tag. The soluble, overexpressed protein was first purified from the crude bacterial lysate by Ni-NTA agarose chromatography. The tag was cleaved by incubation with TEV protease and removed by glutathione agarose chromatography. The protein was purified to homogeneity by two further steps of anion exchange chromatography on a ResourceQ column and gel filtration on a Superdex200 10.30 HR column (Amersham-Pharmacia). The protein was concentrated to 12 mg/ml (0.36micromolar), flash frozen in liquid nitrogen and stored in aliquots at -80° C.

### I.2. Protein Crystallization

Crystals of the RAD51-BRCA2 BRC4 complex were grown in hanging drops by the vapour diffusion method. Drops were prepared by mixing two microliters of protein to two microliters of a 25% ethylene glycol solution, and equilibrated against 750 microliters of the same crystallization solution. Crystals grew at 18°C within a few days to a maximum size of approximately 300×100×100 micrometers. The crystals belong to the space group P2₁2₁2₁ (a = 57.30 Å, b = 59.14 Å, c = 77.20 Å), with one complex in the asymmetric unit.

### I.3. Structure Determination and Refinement

The structure of the RAD51-BRC4 complex was determined using phasing information from SIRAS and MAD experiments. An initial screening by native gel electrophoresis ²⁷ identified KAu(CN)₂ as a potential heavy atom derivative. X-ray data from a native crystal soaked in 0.5mM KAu(CN)₂ for 16 hours were collected to 2 Å resolution. The position of the single gold site was readily determined using direct methods as implemented in Shake 'N' Bake ²⁸. An initial set of phases was calculated with SHARP ²⁹ and improved by the solvent modification routine available within the program. The resulting set of phases were further refined with ARP/WARP 30, which successfully traced the entire chain of the BRC4 repeat and most of the RAD51 ATPase domain. We also prepared selenomethionine-substituted protein that crystallized under the same conditions as the native material. The selenomethionine-containing crystals were used to collect a two-wavelength MAD dataset (peak and high-energy remote at the Se K edge) at station ID29 of the ESRF in Grenoble (France). The MAD phases proved to be of excellent quality and allowed us to extend the resolution of the diffraction data to 1.7 Å and considerably improve our model. Crystallographic refinement was performed using the programs REFMAC ³¹ and CNS ³².

The refined model comprises 1919 protein atoms, 239 water molecules and 4 ethylene glycole molecules. One magnesium ion and one chloride ion were also included in the final model to explain two strong, positive Fₒ-F_{c} difference peaks, located at the carboxyl terminus of the short helix in the initial strand-helix-strand motif, and at the amino terminus of helix A1. Crystallographic data for the structure of the human RAD51-BRCA2 BRC4 complex are summarized in Table 2 (shown in Figure 2), the coordinates of the complex structure are provided in Table 1 (shown in Figure 1), and Figure 4 shows (a) ribbon representations of the RAD51 and BRC4 structures and (b) a schematic diagram of the topology of the complex with numbering of the RAD51 secondary structures (the flexible polypeptide linker being omitted in both (a) and (b)).

237 amino acid residues (98.8%) are in the core region of the Ramachandran plot, 3 in the generously allowed region (1.2%) and none in the disallowed region. RAD51 residues 97, 230 to 236 (loop L1 between beta strand B4 and helix H5), 268 to 292 (loop L2 between strands B5 and B6) and BRCA2 BRC4 residues 1517 to 1518 are not visible in the electron density map and are presumably disordered. The linker joining the BRC repeat to RAD51 is also not detectable in the map, with the exception of the initial ThrGlySer triplet. The quality of the map for the RAD51 region between strands B7 and B8 (residues 316 to 321) is poor, indicating that they are partially disordered in the crystals; the conformation of the polypeptide chain for this loop must therefore be considered tentative. Surface area accessibility calculations were carried out in CNS. Figures were prepared with Molscript ³³ and Raster3D ³⁴.

### I.4. Architecture of the RAD51-BRCA2 BRC4 Complex

The structure of the RAD51-BRCA2 BRC4 complex confirms that RAD51 belongs to the RecA-like family of ATPases (Figure 4), which includes the catalytic subunit of the F1 ATP synthase and the large families of DNA helicases, ABC transporters and the extended AAA-ATPases. RAD51 from Glu98 to its carboxyl terminal residue Asp339 folds into a 3-layer α/β structure with a central, nine-stranded mixed beta sheet (B1 to B9; strand order *987615423)* surrounded by two long, parallel alpha helices (A4 and A5) on one side and three shorter helices (A1 to A3) on the other (Figure 4). The twist angle of the beta sheet increases towards the carboxyl terminus of RAD51, so that the last four antiparallel beta strands can wrap around the amino-terminal strand-loop-strand motif. The ATPase domains of human RAD51 and bacterial RecA ¹⁸ are topologically identical and their superposition results in a root mean square deviation of 1.7Å over 160 Cα atoms (out of 210 present in the crystallographic model).

BRC4 remains in continuous contact with the ATPase domain of RAD51 over a sequence stretch of 28 amino acids (Leu1521 to Glu1548), defining a minimal BRC repeat footprint on RAD51 (Figure 4). Residues Phe1524 to Va11532 fold into a beta hairpin with a 3:5 loop (1526-TASGK-1530) structured as a type I turn followed by a beta bulge at residue Gly1529, which has a positive φ torsion angle 19. The hairpin lines up alongside beta strand B3, thereby extending RAD51's beta sheet by two short anti-parallel strands. After the hairpin, the BRC motif wraps around helix A4 of RAD51 by means of a short linker (residues Lys1533 to Ala1535) that kinks abruptly at residue Lys1536 and leads into an amphipathic alpha-helical segment (residues Lys1536 to Va11542). The remaining residues at the carboxyl end of BRC4 (residues Va11542 to Glu1548) form an irregular coil with elements of a 3₁₀ helix, that spans helices A4 and A5 of RAD51, making an angle of 60° to their axes. Altogether, the BRC motif encircles approximately a third of the hypothetical circumference of RAD51 at its point of maximum diameter.

### 1.5. The RAD51-BRC4 Interface

The RAD51-BRC4 interface is extensive and highly hydrophobic in nature. The total surface area buried during complex formation is 2026 Å². Figure 5(a) shows the solvent-accessible molecular surface of the RAD51 interface superimposed with tube and stick representations of the BRC4 interface residues. The BRC motif is decorated throughout its length with hydrophobic residues that keep it in close contact with RAD51. Three main points of contact stand out, involving the residues Phe1524, Ala 1527 and Phe1546.

Table. 3 (shown in Figure 3) provides a structure-based analysis of BRCA2 BRC sequence conservation and demonstrates that the residues Phe1524, Ala 1527 and Phe1546 are highly conserved in different BRC repeats. In Table 3 the BRC4 sequence from Leu1521 to Glu1548 is displayed horizontally across the top of the table. Residues within elements of secondary structure are boxed. The twenty different amino acids are shown vertically on the left, grouped according to their chemical nature (hydrophilic at the top, hydrophobic at the bottom, the rest in the middle). Each figure in the table indicates the number of times a certain type of amino acid occurs at a particular position in the BRC repeat. The table contains sequence information relative to a set of 56 BRC repeats from 7 different organisms. The information contained in the table is recapitulated by the BRC consensus sequence reported under it ('i' = hydrophobic; 'o' = hydrophilic; 'a' = aromatic, 'x' = no preference).

Phe1524 is located on the strand of the beta hairpin in direct contact with RAD51, and its aromatic ring is completely buried within a hydrophobic cavity formed by the side chains of RAD51 residues Met158 Ile160, Ala190, Ala192, Leu203, Ala207 and Met210. Ala1527, in position L2 of the hairpin loop, places its beta carbon into a small pocket formed by the side chains of RAD51 residues Pro168 Phe166, Leu171, Leu186 and Val189. Phe1546, located in the carboxyl terminal end of the BRC repeat, acts together with Leu1545 to form a wedge embedded between RAD51 helices A4 and A5, and surrounded by residues Leu204, Tyr205, Ser208 (in helix A4) and Met251, Arg254, Leu255, Glu258 and Phe259 (in helix A5). The affinity between BRC4 and RAD51 is further enhanced by hydrophobic contacts involving residues Ile1534 in the linker region, and the hydrogen-bonded Ser1538, Leu1539 and Val1542 in the alpha helix.

Although not as numerous as the hydrophobic interactions, contacts of a polar and charged nature also take place (see Figure 5(b)). The beta hairpin keeps BRC4 in register relative to RAD51 through a set of three continuous, antiparallel main chain-to-main chain hydrogen bonds linking the BRC4 sequence 1525-HTA-1527 to the 190-AYA-192 sequence in strand B3 of RAD51. Asp187 of RAD51 accepts a hydrogen bond from Ser1528, in position L3 of the BRC4 hairpin loop, and interacts electrostatically with Lys1530. Moreover, Glu213 of RAD51 accepts a hydrogen bond from Ser1538 of BRC4, in what is likely to represent a particularly significant contact, because the two side chains are poised for interaction. The position of the Ser1538 side chain is determined by a stacking interaction with BRC4 Ala1535 and RAD51 Val212, while Glu213 is hydrogen bonded to the main chain nitrogen of Ala1535 and, via a water molecule, to the main chain carbonyl of Lys1533. Finally, Glu1548, at the carboxyl end of the BRC4 motif, forms an ion pair with Arg250 of RAD51.

Additional interactions involving residues that are not strongly conserved across BRC repeats help to explain the higher affinity ⁷ of the type 4 repeat towards RAD51 relative to other repeat types. For instance, the tandem repeat of leucine residues 1521 and 1522 are in hydrophobic contact with the side chains of RAD51 residues Phe195 and His199, and the main chain carbonyl of Leu1522 accepts a hydrogen bond from the His199 side chain. His1525 forms a pseudo-hydrophobic core by packing against the aliphatic portions of Lys1535 and Thr1520 side chains and is also hydrogen bonded to the main chain carbonyl of Thr1520, thus conferring further stability to the beta hairpin conformation.

### I.6. A Structure-Based Analysis of BRCA2 BRC Sequence Conservation

The structure of the RAD51-BRC4 complex permits the rationalization of the pattern of sequence conservation displayed by BRC repeats across different repeat types and organisms (Table 3). The most amino-terminal residue to be significantly conserved, Gly1523, is found at a point of secondary structure transition, in a spatially constrained environment at the protein-protein interface. Glycine or serine account for 60% of occurrences at this position, with other less frequent residues being generally of a hydrophobic nature.

Residues 1524-FHTASGK-1530, with the exception of His1525, form a contiguous block of highly conserved amino acids. Phe1524 is the single most conserved BRC residue (present in 89% of the sequences in a set of 56 BRC repeats from seven different organisms): the structure shows that it is involved in a crucial recognition interaction with RAD51. Thr1526 does not contact RAD51, but accepts a hydrogen bond from the main chain nitrogen of Lys1530 that is essential for the conformation of the 3:5 hairpin loop. Thr1526 also donates a hydrogen bond to the hydroxyl function of Ser1528, thus keeping it poised for interaction with RAD51 Asp187. The amino acids threonine or serine account for 93% of occurrences at this position. Like Phe1524, Ala1527 (conserved in 82% of BRC repeats) provides another important point of hydrophobic contact with RAD51. Ser1528 (59%) and Lys1530 (79% preference for a basic residue) are engaged in a polar interaction with Asp187 of RAD51. The preference for a glycine, serine or asparagine (combined frequency of 93%) at position 1529 is dictated by the conformational requirement for a residue that can tolerate a positive φ torsion angle.

Two positions in the linker connecting the beta hairpin to the alpha helix (Va11532 and Ile1534 in BRC4) show a strong preference for aliphatic, branched amino acids (80% and 93% respectively for isoleucine, leucine or valine). The structure demonstrates that Va11532 and Ile1534 contribute to the continuous adherence of the BRC4 motif to the RAD51 surface, through an hydrophobic contact with Met210 of RAD51. Position 1535 marks a point of conformational transition to an alpha helical region, and a serine is found to be prevalent here (with 70% occurrence), likely because of its propensity to cap the helix at its amino terminus.

Within the amphipathic helix, conserved residues including Ser1538 (50% preference) and Leu1539 (89% combined preference for Leu, Ile or Val) make hydrophobic and hydrogen bonded interactions with RAD51. BRC position 1542 shows a clear preference for val, Ala or Ser (79% combined frequency), explained by the structure, where Va11592 marks a point of close contact between BRC4 and helix A4 of RAD51, defining the preference for a small amino acid capable of hydrophobic interaction. However, the strong preference for Lys at positions 1541 (79%) and 1543 (68% combined with arginine) is perplexing because these residues are solvent exposed and do not contact RAD51. Interestingly, Arg rarely occupies position 1541, consistent with a specific role for'lysine, and suggesting that sequence conservation within BRC sequences is not only dictated by their interaction with RAD51.

Leu1545 and Phe1546 in BRC4 are involved in extensive hydrophobic interactions with residues on helices A4 and A5 of RAD51. Indeed, hydrophobic residues are strongly represented at these positions in different BRC repeats (89% and 93% conservation respectively). The structure further demonstrates that, whereas BRC4 residue 1545 is partially solvent exposed, and can therefore accommodate a number of different'side chains, the spatial restraints on residue 1546 are much tighter, as its side chain penetrates deeper into the RAD51-BRC interface. In agreement with our observation, position 1545 shows only a general hydrophobic preference, whereas position 1546 requires either a phenylalanine or a leucine.' The most carboxyl terminal position to show a distinct sequence preference is 1548, which selects for an acidic residue (80% combined conservation for aspartic and glutamic acid). In the crystal structure, Glu1548 forms a salt link with Arg250 of RAD51.

Our analysis shows that the BRC motif is reminiscent of a Velcro strip in the way it adheres to RAD51, that is, through a large number of contacts that are relatively independent from one another. This observation suggests that BRC repeats that differ widely from the consensus, may still retain the capacity to bind RAD51. The elimination of one or a few contact points would weaken the overall binding affinity, without abolishing binding altogether. The BRC sequence might therefore have arisen as a molecular frame suitable for the evolution of amino acid sequences with a wide range of affinities to RAD51, with potential implications for the regulation of RAD51 function by BRCA2.

### I.7. The Human RAD51 Nucleotide-Binding Site

The structure of BRCA2-bound RAD51 reveals some unexpected features of its nucleotide-binding site (see Figures 6(a) and (b)). Lys133 and Thr134, in Walker motif A (127-GEFRTGKT-134), and Asp222, in Walker motif B (218-LLIVD-222), are sequestered in a solvent-inaccessible hydrogen-bonding network that extends to Tyr159, Asp161 and Thr165 via a buried water molecule (Figure 6(a)). Exposed Phe129 at the tip of the phosphate-binding loop (P-loop or Walker motif A) buries part of its aromatic ring in a hydrophobic interaction with Thr134 and Thr165. These contacts do not take place in RecA 18,20, because Lys72 and Thr73 of motif A are further apart from Asp144 in motif B, whereas Glu68 replaces Phe129 in the P-loop. Possibly reflecting the presence of this additional set of interactions, the overall conformation of the P-loop is different in RAD51. A 3-D superposition (Figure 6(b)) shows that, whereas the P-loop remains unchanged in the apo- and ADP-bound forms of RecA 18,20, in BRCA2-bound RAD51 it adopts a more closed conformation that is unlikely to be compatible with its occupation by the ATP phosphates. Although the BRC repeat does not directly mask the ATP-binding site, we speculate that it may cause an indirect conformational effect when bound to RAD51 that inhibits ATP binding.

### I.8. Regulation of RAD51 Nucleoprotein Filament Formation by BRCA2

RAD51 forms helical nucleoprotein filaments on DNA substrates that catalyse pairing and strand exchange between homologous DNA molecules, an essential step in homologous recombination ^{21, 22}. Biological data show that filament formation is abolished when RAD51 is bound to BRC repeat peptides. In *vivo,* over-expression of BRC repeats suppresses the accumulation of RAD51 into nuclear foci after exposure of cells to DNA damaging agents ⁷. *In vitro,* incubation of RAD51 with BRC repeat peptides removes its ability to form nucleoprotein filaments on DNA substrates ¹⁷. Finally, the tendency of RAD51 to spontaneously aggregate into high molecular weight complexes, even in the absence of DNA, is prevented by interaction with BRC repeats, which maintains RAD51 in a monomeric form 17.

The structural basis for filament formation by RAD51 is not known ^{23,24}. In order to gain an insight into the mechanism deployed by BRCA2 to regulate RAD51 filament formation, we analysed the RAD51-BRCA2 interaction in the context of the crystallographic RecA filament (see Figures 7(a) to (d)). In the crystal 18, the RecA molecules pack into a spiral that resembles the nucleoprotein filament formed *in vivo.* Overlaying the RAD51-BRCA2 complex on RecA results in the localization of the BRC beta hairpin at the interface between two adjacent RecA molecules ¹⁸ within the crystallographic filament (Figure 7(a)). Surprisingly, BRC4 residues 1523-GFHTASG-152'9 superimpose closely onto the RecA sequence 25-SIMRLGE-31, which is part of the interface between RecA subunits. RecA residues 27-MRL-29 add in fact an anti-parallel beta strand to the central beta sheet of a neighbouring RecA molecule, in an identical fashion to the interaction of BRC4 residues 1525-HTA-1527 with RAD51 in the RAD51-BRCA2 complex (see Figures 7(b) and (c)).Moreover, RecA residues Ile26 and Leu29 make comparable hydrophobic contacts to those made by Phe1524 and Ala1527 of BRC4 with RAD51.

The superposition analysis provides a strong clue concerning the mechanism adopted by BRCA2 to regulate RAD51 function - BRCA2 binding prevents formation of the nucleoprotein filament by interfering with a crucial contact between RAD51 subunits, and the specific role of the BRC repeats is to mimic the conformation of the RAD51 segment involved in such contact. One prediction of our proposed mechanism is that sequence similarity should be found between the BRC motif and the region of the RAD51 sequence with a putative role in multimerization analogous to that performed by RecA sequence 25-SIMRLGE-31. Indeed, careful inspection of the RAD51 sequence for short motifs resembling the BRC consensus GFxTASG motif identifies the highly conserved sequence 85-GFTTATE-91 in the RAD51 linker between the amino terminal domain and the catalytic core (Figure 7(d)). To test the proposed mechanism, we constructed mutant RAD51 molecules in which amino acids Phe86 and Ala89 within the sequence 85-GFTTATE-91 were replaced by glutamic acid.

### I.9. Formation and Analysis of RAD51 Mutants

Mutant RAD51 molecules (Phe86Glu or Ala89Glu) were fused at their amino terminus to the green fluorescent protein (GFP) reporter before transfection into human cell lines. This was accomplished for each of the Phe86Glu and Ala89Glu mutations by using the QuickChange system (Stratagene) to perform site-directed mutagenesis into a cDNA construct encoding the wild-type RAD51-GFP fusionin pEGFP-C1 (Clontech).

Furthermore, the sequence encoding BRC3 and BRC4 from human BRCA2 was fused at its C-terminus to three consensus nuclear localization signals in the vector pEF-Myc-Nuc (Clontech).

Constructs were verified by nucleotide sequencing. Experiments were carried out 72-96 hrs after transfection of plasmids into 293T cells using the calcium phosphate method. Microscopic images were obtained using a Zeiss LSM510 confocal system equipped with ZeissVision software.

Each of the Phe86Glu and Ala89Glu mutations is predicted to eliminate a critical hydrophobic contact at the RAD51 subunit interface and therefore abolish or significantly weaken RAD51's ability to form filaments.

GFP-RAD51 wild-type, GFP-RAD51 F86E and GFP-RAD51 A89E are expressed at equivalent levels after transfection. As previously observed for endogenous RAD51 ^{25,26}, GFP-RAD51 wild-type accumulates in discrete nuclear foci that represent presumptive sites of DNA damage processing in dividing cells (Figure 8(a)). Formation of these foci is dependent upon RAD51 multimerization, because it is not detected when peptides encoding BRC3 and BRC4 are co-expressed in the same cells (Figure 8(b)); a diffuse nuclear localization of wild-type RAD51 is observed instead, reminiscent of the distribution of GFP alone. Strikingly we find that, when expressed in cells, GFP-RAD51 F86E (Figure 8(c)) and GFP-RAD51 A89E (Figure 8(d)) fail to form foci and are distributed diffusely throughout the nucleus, thus confirming our prediction of an essential role for Phe86 and Ala89 in RAD51 filament formation.

Based on our crystallographic and biological data we therefore conclude that the RAD51 sequence 85-GFTTATE-91 forms an essential part of the interface between RAD51 monomers in the nucleoprotein filament, and residues Phe86 and Ala98 constitute essential points of hydrophobic contact. The sequences 85-GFTTATE-91 in RAD51 and 25-SIMRLGE-31 in RecA mediate a mode of association between subunits that represent a common structural feature of their nucleoprotein filaments.

We further conclude that BRCA2 blocks nucleoprotein filament formation by binding to RAD51 with the BRC consensus sequence GFxTASG, which structurally mimics the RAD51 sequence 85-GFTTATE-91. In the RAD51-BRC4 complex, BRC4 residues Phe1524 and Ala1527 play the same roles that RAD51 residues Phe86 and Ala89 have in the association between RAD51 monomers. The interaction surface between RAD51 and the BRC repeat is more extensive than that provided by the GFxTASG sequence only, as would be expected for a dominant antagonist interaction.

### I.10. Structure-Based Analysis of Cancer-Associated Mutations

Point mutations affecting conserved residues within the BRC repeats predicted to be important for RAD51 binding occur in patients who develop familial breast cancer (Breast Cancer Information Core database, accessible at http::// www.nhgri.nih.gov/Intramural_research/Lab_transfer/Bic/). The common cancer-associated Thr1526 -> Ala mutation impairs the ability of a BRC4 peptide to bind RAD51 ^{7,17}. The structure shows that formation of a hydrogen bond between the hydroxyl function of Thr1526 and the main chain nitrogen of Lys1530 is critical to the conformational integrity of the BRC hairpin loop (Figure 5b). The mutation therefore impairs the affinity of BRCA2 to RAD51 by destabilizing the conformation of the beta hairpin that apposes the BRC repeat to the surface of RAD51. Consistent with the notion that the hydroxyl function mediates an essential interaction, position 1526 is occupied by either a threonine or a serine in 52 out of 56 BRC repeat sequences from seven different organisms (Table 3). BRC repeats in which the threonine is replaced are unlikely to assume the 3:5 hairpin loop conformation required for efficient binding to RAD51. Loss of the critical hydroxyl function at a position analogous to that occupied by Thr1526 in BRC4 has been noted in breast cancer-associated mutations that affect BRC1 (Thr1012 -> Arg) or BRC7 (Thr1981 -> Ile).

Another point mutation associated with familial breast cancer changes Gly1529 in BRC4, at the fourth position of the 3:5 hairpin loop, to arginine. Conformational restraints on position 1529 lead to selection of amino acids able to adopt a positive φ torsion angle, and glycine, serine or asparagine are indeed found in 52 of 56 BRC sequences (Table 2). Replacement of glycine by arginine will disrupt the conformation of the BRC beta hairpin and lead thereby to loss of RAD51 binding capacity.

Thus, structure-based analysis of cancer-associated point mutations affecting the BRC repeats suggests that inheritance of a single alteration that impairs RAD51 binding capacity in just one repeat is enough to cause increased breast cancer susceptibility. One explanation for why the remaining seven BRC repeats should not suffice to preserve function is that the eight BRC repeats present in all vertebrate species work together as a RAD51-binding module whose overall topology is critical for function. For instance, the spacing between individual BRC repeats observed in vertebrate species as evolutionarily distant as chickens and humans is highly conserved. This hints at the possibility ⁹ that interactions with successive BRC repeats in BRCA2 may help to order the distribution of RAD51 molecules in space when, for example, they are being loaded onto substrate DNA during nucleoprotein filament formation, or during removal from established filaments. Alterations that diminish the RAD51 binding capacity of just one of the eight BRC repeats could perturb such functions by interfering with spatial relationships between RAD51 molecules bound to BRCA2.

It has also been suggested that regulation of RAD51 function by BRCA2 may also be modulated by physiological modifications such as phosphorylation ^{9,17}. For instance, phosphorylation of Thr1526 in BRC4 would be predicted to decrease RAD51 binding affinity by destabilising the BRC repeat conformation, whereas phosphorylation of Ser1528 or Ser1538 would disrupt polar contacts with Asp187 or Glu213, respectively, in RAD51. The strong conservation of lysine residues at positions 1541 and 1543 in the helical region of BRC4, which do not make contacts with RAD51, raises the possibility that their solvent exposed amino groups could serve as a target for covalent modifications. From this perspective, we speculate that cancer-associated changes that replace lysine residues corresponding to these conserved positions in BRC1 (Lys1026 ->Glu or Asn) and BRC5 (Lys1691->Asn) may interfere with such events.

Other point mutations in BRCA2 associated with cancer predisposition, such as the frequent change D1420Y near BRC3, fall outside the boundaries of the BRC repeat whose structure we have determined here. An extended BRC3 peptide, which spans the Asp1420 residue, efficiently inhibits nucleoprotein filament formation by RAD51, a property that is abolished in the D1420Y mutant 17. BRCA2 residues outside the BRC consensus sequence defined in this work can therefore additionally contribute to the BRC-RAD51 interaction.

Given that changes in BRCA2 which perturb RAD51 binding give rise to cancer predisposition, our findings raise the possibility that mutations or polymorphisms in RAD51 that impair its interaction with BRCA2 may work in a similar fashion. One reason why such alterations may not yet have been described in breast (or other) cancers is that only a limited number of cases has so far been analysed. Further studies that focus on the prevalence of RAD51 alterations in breast cancers with a familial pattern of incidence may therefore be warranted.

### I.11. A Competitive Inhibition Assay for Screening Substances that disrupt the BRCA2-RAD51 Interaction

The non-oligomerising RAD51 mutants Phe86Glu or Ala89Glu described above can be used in sensitive assays to screen for substances that interrupt the BRCA2-RAD51 interaction, opening an avenue for drug discovery. It would be difficult to perform such assays using wild-type RAD51 because of its tendency to oligomerise spontaneously.

A detailed protocol for the assay follows. Briefly, it involves the coupling of a biotinylated form of a peptide encoding any of the RAD51-binding BRC repeats in BRCA2 (in the example below BRC4 is used) to a strepatavidin-coated matrix suitable for analysis by surface plasmon resonance. The matrix is hereafter termed the "chip".

### I-11.1. Recombinant Protein Purification and Peptide Preparation

pGEX-2TK-RAD51 was constructed by digesting pFB530 with *BamHI* and *Ncol* (New England Biolabs). The resultant RAD51 insert was subjected to a Klenow fill in reaction and sub-cloned via a blunt end ligation into pGEX-2TK (Amersham) digested with *Smal* (New England Biolabs). The non-oligomerising mutant RAD51(F86E) was generated by site-directed mutagenesis of this construct and confirmed by nucleotide sequencing.

Selected E.coli BL21 Codon Plus (Stratagene) transformants were grown overnight at 37°C, in an orbital shaker at 220 rpm in LB medium supplemented with 75µg/ml ampicillin and 50 µg/ml chloranphenicol. The cultures were then diluted 1/20 with LB medium to a final volume of 4L and grown out of selection for 2 hours at 37°C, 220 rpm before induction with 0.1 mM isopropyl-p-D-thiogalactoside (IPTG) and grown further overnight at 22°C. Bacteria were harvested by centrifugation (7,700×g for 20 minutes) and the pellets frozen at -80°C.

For the recovery of GST-RAD51(F86E), the bacterial pellets were resuspended in a total 200 ml phosphate buffered saline (PBS) containing 5 mM dithiothreitol (DTT), 1 mM phenyl methanesulfonylfloride (PMSF) and Complete Protease Inhibitors (PI) (Roche). Following two passes through a French press (1200 psi) and the addition of 1% Triton-X100; the total lysate was incubated for 30 mins at 4°C with rotation. Sample debris and insoluble materials were removed by centrifugation (12,000×g for 60 minutes).

The soluble fraction was applied to 2 ml bed volume of Glutathione Sepherose 4B (GS4B) media (Amersham) and incubated for 4 hours at 4°C with rotation. After protein binding, the coupled media was washed extensively and sedimented by centrifugation (500xg, 5 mins) with three cycles of 50 bed volumes of chilled PBS supplemented with 1 % Triton-X100, 5mM DTT, 1 mM PMSF and PI, followed by three cycles of chilled PBS only.

The Glutathione Sepherose 4B (GS4B) bound GST-RAD51(F86E) fusion protein was cleaved for release from its immobilized GST moiety in 4 m1 PBS for 4h at 25°C using a total of 100U Thrombin protease (Amersham). The flow through elution was collected and subjected to an ion-exchange polishing step.

A 5 ml HiTrap Mono Q FF Anion exchanger (Amersham) was utilized with a flow rate of 4 ml/min and a continuous salt gradient over 20 column volumes (Binding buffer: 10mM Tris pH 7.4, 0.1 M NaCl, Elution buffer: 10mM Tris pH 7.4, 1.1 M NaCl). Peak fractions were pooled and concentrated using a spin column (Viva Spin) before dialysis over night at 4°C against 4L HBS-EP (10 mM HEPES pH 7.4, 0.15M NaCl, 3 mM EDTA, 0.005% v/v Polysorbate 20).

Both biotinylated and unmodified versions of BRC Repeats 1-8 peptides were synthesized and HPLC purified (Cancer Research UK Peptide Synthesis Facility). Expression and purification stages were tracked by the analysis of samples using SDS-PAGE (8-10% acrylamide, Tris-aceate system) and Coomassie Blue staining (BioRad). Concentrations of BRC peptides and recombinant RAD51(F86E) preparations were determined by Bicinchoninic Acid (BCA) assay (Sigma).

### I.11.2. SPR Binding Assay - Ligand Surface Preparation

Measurements were performed on the BIACORE X biosensor (BIACORE AB, Uppsala, Sweden). A continuous flow of de-gassed and 0.22 µm filtered HBSEP running buffer was employed at a constant temperature of 25°C.

Flow cells 1 and 2 of a pre-coated streptavidin Sensor chip SA were conditioned with three sequential 20 µl injections of 1 M NaCl, 50 mM NaOH at a flow rate of 20 µl/min.

Biotinylated BRC Repeat 4 peptide (Bio-BRC4) was immobilized to a level of 200 RU by affinity capture onto Flow cell 2 only. This was achieved by two 20 µl injections of 10 nM Bio-BRC4 in HBS-EP at a flow rate of 20 µl/min. Flow cell 1 represented a reference surface to correct for any refractive index variations and thus was not derivated. Flow cells 1 and 2 were both subsequently treated with three successive injections of 40 µl regeneration solution (10 mM Glycine pH 2.5) at a flow rate of 40 µl/min to ensure baseline stabilization.

The anylate-purified mutant RAD51 Phe86Glu (F86E) or Ala89Glu (A89E) protein in solution can then be run over the BRC repeat coupled-chip in a Biacore instrument (Biacore AB, Uppsala, Sweden) or other suitable measuring device for surface plasmon resonance. Reference corrected data was analysed using BIAevalution software (3.2V) and sensorgrams plotted.

For example, Figure 9(a), which is a sensorgram showing the binding of 5 µM RAD51 F86E in HBSEP to the BRC4 coupled-chip over time, shows that binding can easily be detected, and remains reproducible after successive rounds of chip regeneration. To obtain the results of Figure 9(a), 40 µl of the RAD51 F86E solution was injected over the prepared sensor chip at a flow rate of 40 µl/min, resulting in 60 seconds of contact time followed by 300 seconds of complex dissociation monitoring.

### I.11.3. SPR Binding Assay - Anylate Competition Reactions in Solution

Solutions of 5 µM RAD51 (F86E) in HBS-EP were pre-incubated at 25°C for 30 minutes with or without the addition of the non-biotinylated BRC Repeat peptide (BRC1-8) at various concentrations (0.025 -15 µM) before BIACORE analysis. 40 µl of each solution was then injected over the prepared sensor chip at a flow rate of 40 µl/min, resulting in 60 seconds of contact time followed by 300 seconds of complex dissociation monitoring. Any persisting RAD51 (F86E) was removed by three to four 40 µl injections of regeneration solution (10 mM Glycine pH2.5) at a flow rate of 40 µl/min. To confirm full surface regeneration and the absence of a decaying surface ligand, 5 µM RAD51 (F86E) re-injections were compared over the course of experimentation. This revealed a maximum chip viability of approximately 20 cycles.

Figures 9(b) and (c) are further sensorgrams showing the binding of these 5 µM RAD51 F86E solutions to the BRC4 coupled-chip over time. Figure 9(b) compares the results for different concentrations of the non-biotinylated BRC4, and Figure 9(c) compares the results for different non-biotinylated BRC repeats at the same concentration.

Figure 9(b) shows that pre-incubation of RAD51 F86E mutant protein in the anylate solution with non-biotinylated BRC4 peptide at a 1:1 molar ratio results in an effective inhibition of RAD51 binding. However, at different molar ratios of RAD51 F86E mutant to BRC4 peptide, the dose-dependency of inhibition is apparent. The sensitivity of the assay in detecting competitive inhibition of RAD51 F86E binding by even very small amounts (25 nM, in this example) of BRC4 peptide (i.e., at a 1:200 molar ratio of peptide:RAD51 F86E) is also apparent.

Figure 9(c) shows the different amounts of inhibition of RAD51 F86E binding by peptides encoding other BRC repeats, such as BRC5 or BRC6. This demonstrates the utility of the assay not only in screening for substances that disrupt the BRCA2-RAD51 interaction, but also in comparing their relative potencies.

While the invention has been described in conjunction with the exemplary embodiments described above, many equivalent modifications and variations will be apparent to those skilled in the art when given this disclosure. Accordingly, the exemplary embodiments of the invention set forth are considered to be illustrative and not limiting. Various changes to the described embodiments may be made without departing from the spirit and scope of the invention.

### References

These references, and the other references mentioned above, are hereby incorporated by reference.
1. Nathanson, KN, Wooster, R & Weber, BL. Breast cancer genetics: what we know and what we need. *Nat Med* 7, 552-556. (2001)
2. Bork, P, Blomberg, N & Nilges, M. Internal repeats in the BRCA2 protein sequence [letter]. *Nat Genet* 13, 22-23 (1996)
3. Bignell, G, Micklem, G, Stratton, MR, Ashworth, A & Wooster, R. The BRC repeats are conserved in mammalian BRCA2 proteins. *Hum Mol Genet* 6, 53-58 (1997) .
4. Warren, M, Smith, A, Partridge, N et *al.* Structural analysis of the chicken BRCA2 gene facilitates identification of functional domains and disease causing mutations. *Hum Mol* Genet 11, 841-851. (2002).
5. Wong, AKC, Pero, R, Ormonde, PA, Tavtigian, SV & Bartel, PL. RAD51 interacts with the evolutionarily conserved BRC motifs in the human breast cancer susceptibility gene brca2. J *Biol Chem* 272, 31941-31944 (1997).
6. Chen, PL, Chen, CF, Chen, Y et *al.* The BRC repeats in BRCA2 are critical for RAD51 binding and resistance to methyl methanesulfonate treatment. *Proc Natl Acad Sci U S A* **95,** 5287-5292 (1998).
7. Chen, CF, Chen, PL, Zhong, Q, Sharp, ZD & Lee, WH. Expression of BRC repeats in breast cancer cells disrupts the BRCA2- Rad51 complex and leads to radiation hypersensitivity and loss of G(2)/M checkpoint control. *J Biol Chem* **274,** 32931-32935 (1999).
8. Scully, R & Livingston, DM. In search of the tumour-suppressor functions of BRCA1 and BRCA2. *Nature* 408, 429-432. (2000).
9. Venkitaraman, AR. Cancer susceptibility and the functions of BRCA1 and BRCA2. *Cell* 108, 171-182. (2002).
10. Chen, G, Yuan, SS, Liu, W *et al.* Radiation-induced assembly of Rad51 and Rad52 recombination complex requires ATM and c-Abl. *J Biol Chem* **274**, 12748-12752 (1999).
11. Yu, VPCC, Koehler, M, Steinlein, C *et al*. Gross chromosomal rearrangements and genetic exchange between non-homologous chromosomes following BRCA2 inactivation. *Genes Dev* 14, 1400-1406 (2000).
12. Moynahan, ME, Pierce, AJ & Jasin, M. BRCA2 is required for homology-directed repair of chromosomal breaks. *Mol Cell* 7, 263-272. (2001).
13. Lim, DS & Hasty, P. A mutation in mouse rad51 results in an early embryonic lethal that is suppressed by a mutation in p53. *Mol Cell Biol* 16, 7133-7143 (1996).
14. Tsuzuki, T, Fujii, Y, Sakumi, K *et al.* Targeted disruption of the Rad51 gene leads to lethality in embryonic mice. *Proc Natl Acad Sci U S A* 93, 6236-6240 (1996).
15. Patel, KJ, Yu, VPCC, Lee, H et *al*. Involvement of Brca2 in DNA repair. *Mol Cell* 1, 347-357 (1998).
16. Tutt, A, Gabriel, A, Bertwistle, D *et al*. Absence of brca2 causes genome instability by chromosome breakage and loss associated with centrosome amplification. *Curr Biol* **9**, 1107-1110 (1999).
17. Davies, AA, Masson, JY, McIlwraith, MJ et a1. Role of BRCA2 in control of the RAD51 recombination and DNA repair protein. *Mol Cell* 7, 273-282. (2001).
18. Story, RM, Weber, IT & Steitz, TA. The structure of the E. coli recA protein monomer and polymer. Nature 355, 318-325. (1992).
19. Sibanda, BL & Thornton, JM. Beta-hairpin families in globular proteins. *Nature* 316, 170-174. (1985).
20. Story, RM & Steitz, TA. Structure of the recA protein-ADP complex. *Nature* 355, 374-376. (1992).
21. Sung, P & Robberson, DL. DNA strand exchange mediated by a RAD51-ssDNA nucleoprotein filament with polarity opposite to that of RecA. *Cell* 82, 453-461 (1995).
22. Baumann, P, Benson, FE & West, SC. Human Rad51 protein promotes ATP-dependent homologous pairing and strand transfer reactions in vitro. *Cell* 87, 757-766. (1996).
23. Ogawa, T, Yu, X, Shinohara, A.& Egelman, EH. Similarity of the yeast RAD51 filament to the bacterial RecA filament. *Science* 259, 1896-1899. (1993).
24. Yu, X, Jacobs, SA, West, SC, Ogawa, T & Egelman, EH. Domain structure and dynamics in the helical filaments formed by RecA and Rad51 on DNA. *Proc Natl Acad Sci USA* 98, 8419-8424. (2001) .
25. Haaf, T, Golub, EI, Reddy, G, Radding, CM & Ward, DC. Nuclear foci of mammalian Rad51 recombination protein in somatic cells after DNA damage and its localization in synaptonemal complexes. *Proc Natl Acad Sci USA* **92,** 2298-2302 (1995).
26. Scully, R, Chen, J, Plug, A et *al.* Association of BRCA1 with Rad51 in mitotic and meiotic cells. *Cell* 88, 265-275. (1997).
27. Boggon, TJ & Shapiro, L. Screening for phasing atoms in protein crystallography, Structure *Fold Des* 8, R143-149. (2000).
28. Deacon, AM, Weeks, CM, Miller, R & Ealick, SE. The Shake- and-Bake structure determination of triclinic lysozyme. *Proc Natl Acad Sci USA* 95, 9284-9289. (1998).
29. La Fortelle, E & Bricogne, G. Maximum-likelihood heavy-atom parameter refinement in the MIR and MAD methods. Methods *Enzymol* 276, 472-294 (1997).
30. Perrakis, A, Morris, R & Lamzin, VS. Automated protein model building combined with iterative structure refinement. *Nat Struct Biol* 6, 458-463. (1999).
31. Murshudov, GN, Vagin, AA & Dodson, EJ. Refinement of Macromolecular Structures by the Maximum-Likelihood Method. Acta *Crystallogr. D* 54, 905-921 (1997).
32. Bruenger, A. *Crystallography & NMR system:* a new software suite for macromolecular structure determination. *Acta Crystallogr. D 54,* 905-921 (1998).
33. Kraulis, PJ. MOLSCRIPT: a program to produce both detailed and schematic plots of protein structures. *J. Appl. Cryst.* 24(1991).
34. Merritt, EA & Bacon, DJ. Raster3D photorealistic molecular graphics. *Meth. Enzymol.* 277, 505-524 (1997).

## Claims

1. A mutant RAD51 which has been modified to reduce or eliminate the tendency of RAD51 to spontaneously aggregate into high molecular weight complexes.

2. A mutant RAD51 which has been modified by substitution, deletion and/or addition of at least one amino acid in the 85-GFTTATE-91 sequence of human RAD51, or the corresponding sequence in other forms of RAD51.

3. A nucleic acid encoding the mutant RAD51 of claim 1 or 2.

4. Use of the mutant RAD51 of claim 1 or 2 in an assay for identifying compounds which interact with or form part of a RAD51 pathway.

5. A compound which is identified by the method of claim 4.

6. A crystal of a RAD51-BRC repeat sequence complex.

7. A crystal according to claim 6 having the orthorhombic space group P2₁2₁2₁, and unit cell dimensions a = 57.30 Å ± 5%, b = 59.14 Å ± 5%, c = 77.20 Å ± 5%.

8. A crystal according to claim 6 which diffracts X-rays for the determination of atomic coordinates of the complex to a resolution of better than 2.0 Å.

9. A crystal according to claim 6 having the three dimensional atomic coordinates of Table 1.

10. A RAD51-BRC repeat sequence chimaera protein in which the RAD51 is covalently joined to the BRC repeat sequence.

11. A RAD51 paralogue-BRC repeat sequence chimaera protein in which the RAD51 paralogue is covalently joined to the BRC repeat sequence.

12. A nucleic acid encoding the chimaera protein of claim 10 or 11.

13. A method of homology modelling comprising the steps of:
(a) aligning a representation of an amino acid sequence of a target protein of unknown three-dimensional structure with the amino acid sequence of the RAD51 or the BRC repeat sequence of Table 1 to match homologous regions of the amino acid sequences;
(b) modelling the structure of the matched homologous regions of said target protein of unknown structure on the corresponding regions of the RAD51 or BRC repeat sequence structure as defined by Table 1; and
(c) determining a conformation for said target protein of unknown structure which substantially preserves the structure of said matched homologous regions.

14. A method for determining the structure of a protein, which method comprises;
providing the co-ordinates of Table 1, and
positioning the co-ordinates in the crystal unit cell of said protein so as to provide a structure for said protein.

15. A method for determining the structure of a compound bound to RAD51 or a BRC repeat sequence, said method comprising:
providing a crystal of a complex in which a compound is bound to RAD51 or a BRC repeat sequence; and
determining the structure of said complex by employing the data of Table 1.

16. A computer-based method for the analysis of the interaction of a molecular structure with RAD51 or BRC repeat sequence, which comprises:
providing the structure of RAD51 or a BRC repeat sequence as defined by Table 1;
providing a molecular structure to be fitted to said RAD51 or BRC repeat sequence structure; and
fitting the molecular structure to the RAD51 or BRC repeat sequence structure.

17. A computer-based method for the analysis of the interaction of a molecular structure with RAD51 or BRC repeat sequence, which comprises:
providing the coordinates of at least two atoms of RAD51 or a BRC repeat sequence structure as defined by Table 1;
providing a molecular structure to be fitted to said coordinates; and
fitting the structure to the said coordinates.

18. A method of determining the biological activity of a compound, which comprises:
identifying a compound which fits to RAD51 or a BRC repeat sequence by performing the method of claim 16 or 17;
obtaining or synthesizing the compound; and
testing the compound in an *in vivo* or *in vitro* biological system in order to determine the activity of the compound.

19. A compound which is identified by the method of claim 16 or 17.
